# EUROPEAN PATENT APPLICATION

(11) **EP 4 424 841 A1**
(43) Date of publication of application: **04.09.2024**
(21) Application number: 23159425.0
(22) Date of filing: 01.03.2023
(51) Int. Cl.: C12Q 1/6834, C12Q 1/6839

(54) **MEDIATING THE BINDING OF PAYLOADS TO SURFACES USING NUCLEIC ACID NANOSTRUCTURES**

(71) Applicant: Nanogami GmbH, 04103 Leipzig (DE)
(72) Inventor: Sobczak, Jean-Philippe Jacques, 81671 Munich (DE); Patil, Sumersing Virendrasing, 81671 Munich (DE); Aigner-Bogner, Tamara Bernadette, 81671 Munich (DE); Aghebat Rafat, Ali, 81671 Munich (DE)
(74) Representative: Fish & Richardson P.C.

(57) **Abstract**

The disclosure relates to systems and methods for binding payload moieties to a surface. In particular, the binding is mediated by nucleic acid nanostructures.

## Description

### Field

The disclosure relates to systems and methods for binding payload moieties to a surface. In particular, the binding is mediated by nucleic acid nanostructures.

### Background

In a variety of different contexts, it can be desirable to control the position and/or environment of molecules. Example contexts include analytical contexts (e.g., proteomics, sequencing, sensing), chemical syntheses, nanomachines, data storage, photonics, quantum computing, and electronics. However, controlling the placement on a molecular level is difficult.

Nucleic acid nanostructures can be assembled using DNA origami and related techniques. With proper selection of the constituent sequences of bases, strands of nucleic acids can fold into desired shapes. Software can be used to design the nucleic acid nanostructures.

### Summary

The disclosure relates to systems and methods for binding payload moieties to a surface. The binding is mediated by nucleic acid nanostructures. The systems and methods can maintain a minimum distance between payload moieties on a surface.

In this context, payload moieties are moieties (e.g., molecules) relevant in an analytical context (e.g., proteomics, sequencing, sensing), chemical syntheses, nanomachines, data storage, photonics, quantum computing, and/or electronics. Examples of payload moieties include an enzyme, a protein, a fluorescent dye, a quencher, a quantum dot, a nanoparticle, a nucleic acid, a chemical linker group, a coordination complex, and a molecular switch.

The systems and methods for binding payload moieties to a surface use nucleic acid nanostructures. The size, shape and/or chemical composition of the nucleic acid nanostructures are relatively addressable. The nucleic acid nanostructures can be placed on a surface in a relatively controllable manner. The surfaces do not have to be tailored for binding either the nucleic acid nanostructures or the payload moieties. Rather, the placement of payload moieties on the surface is mediated by the size, shape and/or chemical composition of the nucleic acid nanostructures. The nucleic acid nanostructures provide a minimum distance between payload moieties and position a controllable number of payload moieties in controlled relative orientations and locations to each other in an area of the surface. The systems and methods of the disclosure can reduce or prevent payload moieties from being too close together on the surface.

The payload moieties can be positioned at sites of the nucleic acid nanostructures. As discussed further below, the sites can present the payload moieties on surfaces of the nucleic acid nanostructures such that they are sterically-accessible. Alternatively, the sites can sterically hinder access to the payload moieties, e.g., the payload moieties can be sited within the nucleic acid nanostructures. Because the shapes of nucleic acid nanostructures are structured by the complementary binding of base pairs, the sites of the nucleic acid nanostructures with payload moieties can be reproducibly tailored to achieve a precise and reproducible placement of the payload moieties on or in the nucleic acid nanostructures. Different nucleic acid nanostructures and/or payload moieties can be designed, as needs be.

In a first aspect, the disclosure provides a method including exposing a plurality of nucleic acid nanostructures to a surface functionalized with a plurality of first moieties, each of the nucleic acid nanostructures including a payload moiety positioned at a site in each of the nucleic acid nanostructures and a second moiety; and forming either a covalent bond or a ligand-receptor interaction between the first moieties and the second moieties such that positions of the nucleic acid nanostructures and the payload moieties are constrained relative to the surface. The sites of the payload moieties in the nucleic acid nanostructures are disposed such that a minimum distance is maintained between the payload moieties of each nucleic acid nanostructure when the first moieties covalently bond or interact via the ligand-receptor interaction with second moieties.

In some embodiments, the second moieties are coupled to a generally planar face of the nucleic acid nanostructures, and the nucleic acid nanostructures have a height dimension extending perpendicularly from the generally planar face that is smaller than other dimensions of the nucleic acid nanostructures.

In some embodiments, the nucleic acid nanostructures include a length of 10 nm to 1000 nm. In some embodiments, the nucleic acid nanostructures include a height of 2 nm to 1000 nm. In some embodiments, a ratio of length to height of the nucleic acid nanostructures is 1:1 to 500:1. In some embodiments, a height dimension of the nucleic acid nanostructures is less than 20% of the smallest of the other dimensions of the nucleic acid nanostructures.

In some embodiments, the payload moieties are coupled to the nucleic acid nanostructures in a vicinity of a middle of the other dimensions of the nucleic acid nanostructures.

In some embodiments, each nucleic acid nanostructures includes a first face and a second face. The second moiety is disposed on the first face. The payload moiety is disposed on the second face.

In some embodiments, each nucleic acid nanostructure further includes a first portion including a nucleic acid including the second moiety and the payload moiety, and the method further includes removing a second portion of each nucleic acid nanostructure from the surface. The second portion is different from the first portion.

In some embodiments, each nucleic acid nanostructure includes a cleavable group and the second moiety and the payload moiety are attached to the nucleic acid nanostructure via the cleavable group, and the method further includes cleaving the cleavable groups.

In some embodiments, the nucleic acid nanostructures include a flexible linker and the second moiety is attached to the flexible linker.

In some embodiments, the surface is flat. In some embodiments, the surface is unpatterned. In some embodiments, the surface includes depressions functionalized with the first moiety and the depressions are configured such that at most one nucleic acid nanostructure can occupy each depression.

In some embodiments, the payload moiety includes an enzyme, a protein, a fluorescent dye, a quencher, a quantum dot, a nanoparticle, a nucleic acid strand, a chemical linker group, a coordination complex, and/or a molecular switch. In some embodiments, the payload moiety includes a protein selected from the group consisting of streptavidin, a streptavidin-like moiety, a polymerase, an antibody, horseradish peroxidase, a protein from a patient sample, a G protein, a G protein-coupled receptor (GPCRs), an ion channel, a cell-surface protein, and/or a receptor protein. In some embodiments, the payload moiety includes a nucleic acid selected from the group consisting of DNA, RNA, a viral sequence, a human sequence, a bacterial sequence, an artificial sequence and/or a sequence with non-natural bases.

In some embodiments, a minimum distance between payload moieties of each nucleic acid nanostructures is at least 10 nm. In some embodiments, a minimum distance between payload moieties of each nucleic acid nanostructures is at least 100 nm. In some embodiments, a minimum distance between payload moieties of each nucleic acid nanostructures is at least 1000 nm.

In some embodiments, a density of the nucleic acid nanostructures is 1 to 10000 per µm². In some embodiments, a density of payload moieties is 1 to 1000000 per µm².

In a second aspect, the disclosure provides a system, including a surface functionalized with a plurality of first moieties; and a plurality of nucleic acid nanostructures, each of the nucleic acid nanostructures including a payload moiety, and a second moiety that covalently bonds or interacts via a ligand-receptor interaction with the first moieties. The positions of the nucleic acid nanostructures and the payload moieties are constrained relative to the surface. The nucleic acid nanostructures are configured on the surface such that a minimum distance is maintained between the payload moieties of each nucleic acid nanostructures.

In a third aspect, the disclosure provides a method including performing proteomics, sequencing, super-resolution microscopy, protein analytics, surface plasmon resonance (SPR), Bio-layer interferometry (BLI), switchSENSE, quantum computing, photonics, light harvesting, catalysts, nucleic acid data storage, and/or chemical sensing, using a system of the disclosure.

In a fourth aspect, the disclosure provides a system, formed by a method of the disclosure.

### Brief Description of the Figures

FIG. 1 depicts a schematic of a method.
FIG. 2 depicts a schematic of a method.
FIG. 3A depicts a schematic of a nucleic acid nanostructure.
FIG. 3B depicts a schematic of a nucleic acid nanostructure unbound and bound to a surface.
FIG. 4 depicts a schematic of a method.
FIG. 5 depicts a schematic of a method.
FIG. 6 depicts a schematic of a method.
FIG. 7 depicts a schematic of a method.
FIG. 8 depicts a schematic of a method.
FIG. 9 depicts a schematic of a method.
FIG. 10A depicts a schematic of a method.
FIG. 10B depicts a schematic of a method.
FIG. 10C depicts a schematic of a method.
FIG. 10D depicts a schematic of a method.
FIG. 11 depicts a schematic of a method.
FIG. 12A depicts a schematic of a method.
FIG. 12B depicts a schematic of a method.
FIG. 13A depicts a schematic of a method.
FIG. 13B depicts a schematic of a method.
FIG. 14 depicts a schematic of a nucleic acid nanostructure.
FIG. 15 depicts a schematic of a nucleic acid nanostructure and first and second binding configurations of the nucleic acid nanostructure to a surface.
FIG. 16 depicts a schematic of a nucleic acid nanostructure and first and second binding configurations of the nucleic acid nanostructure to a surface.
FIG. 17 shows a fluorescent image of nucleic acid nanostructures covalently bound to a functionalized surface.
FIG. 18 shows a fluorescent image of nucleic acid nanostructures covalently bound to a functionalized surface.
FIG. 19 shows a fluorescent image of nucleic acid nanostructures covalently bound to a functionalized surface.
FIG. 20 shows a fluorescent image of nucleic acid nanostructures covalently bound to a functionalized surface.
FIG. 21 shows a fluorescent image of nucleic acid nanostructures covalently bound to a functionalized surface.
FIG. 22 shows a fluorescent image of nucleic acid nanostructures covalently bound to a functionalized surface.
FIG. 23 a graph of the probability of a second payload moiety not being placed relative to a first payload moiety as a function of distance.

### Detailed Description

### Disposal of payload-bearing nucleic acid nanostructures on a surface

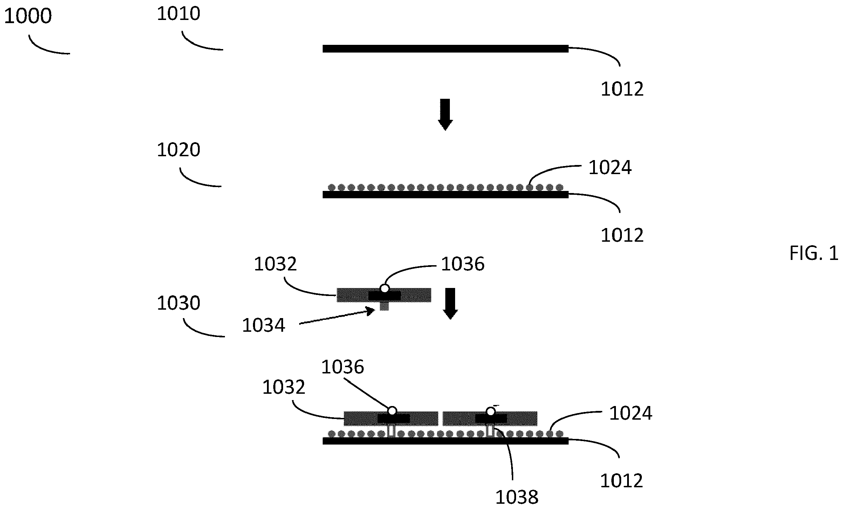

FIG. 1 depicts a schematic for a method 1000. In step 1010, a surface 1012 is provided. For example, the surface 1012 can be a glass, quartz, fused silica, silicon, silicon oxide, gold, aluminum, graphene, glass, and/or a polymer surface. In step 1020, the surface 1012 is functionalized with a first moiety 1024. The first moiety 1024 can be, for example, a chemical group that is a reactant in a click chemistry reaction or biotin.

In step 1030, a plurality of nucleic acid nanostructures 1032 are disposed on the surface 1012. Nucleic acid nanostructures 1032 can be DNA origami nanostructures with shapes that are constrained by interactions between complementary bases. In the illustrated implementations, all of the nucleic acid nanostructures 1032 are shown as generally rectangular cuboid structures with a smaller height dimension (upward/downward in the plane of the page) and larger length and width dimensions (leftward/rightward in the plane of the page and into/out the plane of the page). For example, the nucleic acid nanostructures can have a length and/or width of 10-1000 nm, a height of 2-1000 nm, and/or a ratio of length and/or width to height of 1: 1 to 500:1.

In other implementations, nucleic acid nanostructures 1032 need not have rectangular cuboid shapes. Rather, the nucleic acid nanostructures 1032 can have a circular, spherical, square, cuboidal, triangular, pyramidal, rod-like or ring-like structures.

The nucleic acid nanostructures 1032 include one or more second moieties 1034 and a payload moiety 1036. The second moieties 1034 are configured to interact complementarily with the first moieties 1024. For example, the first moieties 1024 and second moieties 1034 can form one or more covalent bonds, ligand-receptor interaction(s), and/or van der Waals interactions. The second moieties 1034 are generally covalently bound or attached via ligand-receptor (e.g., biotin-streptavidin) interactions to nucleic acid nanostructures 1032 at sterically-accessible positions such that the second moieties 1034 can interact with first moieties 1024 when nucleic acid nanostructures 1032 are in the vicinity of surface 1012. The second moieties 1034 can be for example, a chemical group that is a reactant in a click chemistry reaction or biotin. In some embodiments, each nucleic acid nanostructure 1032 includes more than one second moiety 1034. In some embodiments, each nucleic acid nanostructure 1032 includes at least 1 (e.g., at least 2, at least 3, at least 4, at least 5, at least 6, at least 7, at least 8, at least 9, at least 10, at least 50 at least 100, at least 1000, at least 10000, at least 100000) and/or at most 250000 (e.g., at most 100000, at most 10000, at most 1000, at most 100, at most 50, at most 10) second moieties 1034.

As discussed above, payload moieties 1036 can be a moiety (e.g., a molecule) relevant in an analytical context (e.g., proteomics, sequencing, sensing), chemical syntheses, nanomachines, data storage, photonics, quantum computing, and/or electronics. In some implementations, payload moieties 1036 and second moieties 1034 are generally bound to opposite sides of nucleic acid nanostructures 1032 such that when second moieties 1034 interact with first moieties 1024 at a surface, payload moieties 1036 are sterically accessible from the other side of nucleic acid nanostructures 1032. In other implementations, payload moieties 1036 are bound within nucleic acid nanostructures 1032 and sterically inaccessible. For example, payload moieties 1036 can be an enzyme, a protein, a fluorescent dye, a quencher, a quantum dot, a nanoparticle, a nucleic acid, a chemical linker group, a coordination complex, or a molecular switch.

The complementary interactions between the first moieties 1024 and second moieties 1034 constrain the positions of the nucleic acid nanostructures 1032 and the payload moieties 1036 relative to the surface 1012. With the known positioning of payload moieties 1036 in nucleic acid nanostructures 1032 and the steric blocking provided by nucleic acid nanostructures 1032, a minimum distance (e.g., at least 10 nm, at least 100 nm, at least 1000 nm) can be maintained between the payload moieties 1036.

The shape of the nucleic acid nanostructures 1032 creates a minimum distance between the payload moieties 1036 so long as nucleic acid nanostructures 1032 do not overlap on the surface 1012. In some embodiments, the minimum distance between payload moieties 1036 is at least 10 (e.g., at least 20, at least 30, at least 40, at least 50, at least 60, at least 70, at least 80, at least 90, at least 100, at least 150, at least 200, at least 250, at least 300, at least 350, at least 400, at least 450, at least 500, at least 550, at least 600, at least 650, at least 700, at least 750, at least 800, at least 850, at least 900, at least 950) nm and/or at most 1000 (e.g., at most 950, at most 900, at most 850, at most 800, at most 750, at most 700, at most 650, at most 600, at most 550, at most 500, at most 450, at most 400, at most 350, at most 300, at most 250, at most 200, at most 150, at most 100, at most 90, at most 80, at most 70, at most 60, at most 50, at most 40, at most 30, at most 20) nm. The size and shapes of the nucleic acid nanostructures 1032 are discussed below.

In certain embodiments, the density of the nucleic acid nanostructures 1032 on the surface 1012 is at least 1 (e.g., at least 10, at least 100, at least 1000) per µm² and/or at most 10000 (e.g., at most 1000, at most 100, at most 10) per µm². In certain embodiments, the density of payload moieties 1036 on the surface 1012 is at least 1 (e.g., at least 10, at least 100, at least 1000, at least 10000, at least 100000) per µm² and/or at most 1000000 (e.g., at most 100000, at most 10000, at most 1000, at most 100, at most 10) per µm². In certain embodiments, a minimum distance between the centers of adjacent nucleic acid nanostructures 1032 on the surface 1012 is at least 10 (e.g., at least 50, at least 100, at least 500) nm and/or at most 1000 (e.g., at most 500, at most 100, at most 50) nm.

In some embodiments, the proportion of nucleic acid nanostructures 1032 having a payload moiety 1036 and a desired up-down orientation on the surface 1012 is at least 51 (e.g., at least 60, at least 70, at least 80, at least 90, at least 95, at least 98, at least 99, at least 99.5, at least 99.8, at least 99.9) % and/or at most 100 (e.g., at most 99.9, at most 99.8, at most 99.5, at most 99, at most 98, at most 95, at most 90, at most 80, at most 70, at most 60) %.

Generally, the surface 1012 can be functionalized with the first moiety 1024 in the step 1020 using any appropriate method and reagent. In some embodiments, the surface 1012 is functionalized with (3-Mercaptopropyl)triethoxysilane, (3-Glycidyloxypropyl)trimethoxysilane, 3-(Trimethoxysilyl)propylmethacrylate, Cyanomethyl [3-(trimethoxysilyl)propyl] trithiocarbonate, 3-Aminopropyltriethoxysilane (APTES), and/or Azide-PEG-Silane.

In some embodiments, the complementary interaction 1038 between first moieties 1024 and second moieties 1034 is a covalent bond, such as a covalent bond formed from a click-chemistry reaction. In some embodiments, the complementary interaction 1038 is formed by a reaction between an azide group and an alkyne group (e.g., a reaction between an azide group and DBCO), a reaction between a thiol group and an alkene group, a reaction between a thiol group and a maleimide group, and/or a reaction between a first thiol group and a second thiol group. In some embodiments, the complementary interaction 1038 is formed by a reaction between an amine group and an NHS-ester group, an activated carboxylic acid group, an epoxy group, an isothiocyanate (R-N=C=S) group, an acyl chloride, and/or an acid anhydride. In some embodiments, the complementary interaction is formed by a reaction between an epoxide group and an amide group, an alcohol group, a thiol group and/or a carboxylic acid group. The first moiety 1024 and the second moiety 1034 can be selected as appropriate for the desired reaction.

In some embodiments, the first moiety 1024 includes an amine group and the second moiety 1034 includes an N-hydroxysulfosuccinimide group. In some embodiments, the first moiety 1024 includes an N-hydroxysulfosuccinimide group and the second moiety 1034 includes an amine group. In some embodiments, the first moiety 1024 includes an amine group and the second moiety 1034 includes a carboxylic acid group. In some embodiments, the first moiety 1024 includes a carboxylic acid group and the second moiety 1034 includes an amine group. In some embodiments, the first moiety 1024 includes a maleimide group and the second moiety 1034 includes a thiol group. In some embodiments, the first moiety 1024 includes a thiol group and the second moiety 1034 includes a maleimide group. In some embodiments, the first moiety 1024 includes a thiol group and the second moiety 1034 includes a thiol group. In some embodiments, the first moiety 1024 includes a thiol group and the second moiety 1034 includes an alkene or alkyne group. In some embodiments, the first moiety 1024 includes an alkene or alkyne group and the second moiety 1034 includes a thiol group.

In some embodiments, the complementary interaction 1038 is a chelation interaction, such as an interaction between a Ni²⁺-NTA group and a histidine tag. In some embodiments, the first moiety 1024 includes a Ni²⁺-NTA group and the second moiety 1034 includes a histidine tag. In some embodiments, the first moiety 1024 includes a histidine tag and the second moiety 1034 includes a Ni²⁺-NTA group.

In certain embodiments, the complementary interaction 1038 is a gold thiol interaction. In certain embodiments, the first moiety 1024 includes gold (e.g., the surface 1012 is coated with gold) and the second moiety 1034 includes a thiol group. In certain embodiments, the first moiety 1024 include a thiol group and the second moiety 1034 includes a gold nanoparticle.

In some embodiments, the complementary interaction 1038 is formed by a reversible addition-fragmentation chain-transfer (RAFT) polymerization reaction. In some embodiments, the RAFT polymerization is performed with a RAFT agent with a trithiocarbonate group (e.g. Cyanomethyl [3-(trimethoxysilyl)propyl] trithiocarbonate); a functional monomer, such as a moiety with a vinyl group (e.g. butadiene), an aromatic ring (e.g. styrene), a carbonyl group (e.g. (meth)acrylate), and/or a nitrile group (e.g. acetonitrile); and a chain transfer agent (e.g. a trithiocarbonate, a dithiobenzoate).

In certain embodiments, the complementary interaction 1038 is formed by an acrylate radical polymerization. In certain embodiments, the acrylate radical polymerization is performed with an acrylate bearing moiety (e.g. 3-(Trimethoxysilyl)propylmethacrylate) and an acrylate bearing moiety (e.g. methyl acrylate, methyl methacrylate, 2-ethyl hexyl acrylate).

In some embodiments, the complementary interaction 1038 is a ligand-receptor interaction. In some embodiments, the ligand-receptor interaction is an interaction between biotin and streptavidin or a streptavidin-like moiety (e.g. avidin, neutravidin, captavidin). In some embodiments, the first moiety 1024 includes biotin and the second moiety 1034 includes streptavidin or a streptavidin-like moiety. In some embodiments, the first moiety 1024 includes streptavidin or a streptavidin-like moiety and the second moiety 1034 includes biotin. In some embodiments, the first moiety 1024 and the second moiety 1034 include biotin, and streptavidin or a streptavidin-like moiety is introduced prior to introducing the nucleic acid nanostructures 1032.

In some embodiment, the complementary interaction 1038 is an antibody-antigen interaction. In some embodiments, the first moiety 1024 includes an antigen and the second moiety 1034 includes an antibody capable of binding the antigen. In some embodiments, the second moiety 1034 includes an antigen and the first moiety 1024 includes an antibody capable of binding the antigen.

In certain embodiments, the complementary interaction 1038 is a non-covalent interaction. In certain embodiments, the non-covalent interaction can be mediated by DNA-DNA interactions, ions, and/or charged polymers. For example, in certain embodiments, the surface 1012 includes polylysine and the nucleic acid nanostructures 1032 will bind to the surface 1012 non-covalently due to the positive charge of the polylysine. As another example, in certain embodiments, the surface 1012 includes a hydrogel or polymer with negatively charged moieties (e.g., carboxyl groups) and binding of the nucleic acid nanostructures 1032 to the surface 1012 is mediated by a multivalent ion such as Mg²⁺.

The position and number of the payload moiety 1036 on the nucleic acid nanostructure 1032 can be controlled. For example, each nucleic acid nanostructure 1032 can include a single payload moiety 1036. Thus, the binding of a nucleic acid nanostructure 1032 to the surface 1012 positions one or more payload moieties 1036 relative to the surface 1012. In certain embodiments, each nucleic acid nanostructure 1032 includes more than one payload moiety 1036 (see discussion below). In certain embodiments, the payload moiety 1036 is coupled to the nucleic acid nanostructure 1032 in a vicinity of a middle of the other dimensions of the nucleic acid nanostructure.

Examples of the payload moiety 1036 include an enzyme, a protein, a fluorescent dye, a quencher, a quantum dot, a nanoparticle, a nucleic acid, a chemical linker group, a coordination complex, and a molecular switch. Examples of a protein as the payload moiety 1036 include streptavidin, a streptavidin-like moiety, a polymerase, an antibody, horseradish peroxidase, a protein from a patient sample, a G protein, a G protein-coupled receptor (GPCRs), an ion channel, a cell-surface protein, and a receptor protein. Examples of a nucleic acid as the payload moiety 1036 include DNA, RNA, a viral sequence, a human sequence, a bacterial sequence, an artificial sequence and/or a sequence with one or more non-natural bases.

The step 1030 can include disposing a solution containing the nucleic acid nanostructures 1032 onto the surface 1012. In certain embodiments, a concentration of the nucleic acid nanostructures 1032 in a solution disposed onto the surface 1012 is at least 1 pM (e.g., at least 10 pM, at least 100 pM, at least 1 nM, at least 10 nM, at least 100 nM) and/or at most 1 µM (e.g., at most 100 nM, at most 10 nM, at most 1 nM, at most 1 nM, at most 100 pM, at most 10 pM).

Examples of the surface 1012 include glass, quartz, fused silica, silicon, silicon oxide, gold, aluminum, graphene, glass, and/or a polymer such as PDMS, PMMA, polystyrene, a cyclic olefin copolymer (COC), a polycarbonate, and/or a polypropylene polymer (e.g. a nylon membrane). In some embodiments, the surface 1012 is passivated to reduce non-specific interactions. In some embodiments, the passivation of the surface 1012 includes PEG chains, 3D polymer matrices and/or 3D hydrogels. In some embodiments, the surface 1012 is homogenous in that it does not include patterns, distinct regions and/or structures to mediate the binding of the nucleic acid nanostructures to the surface.

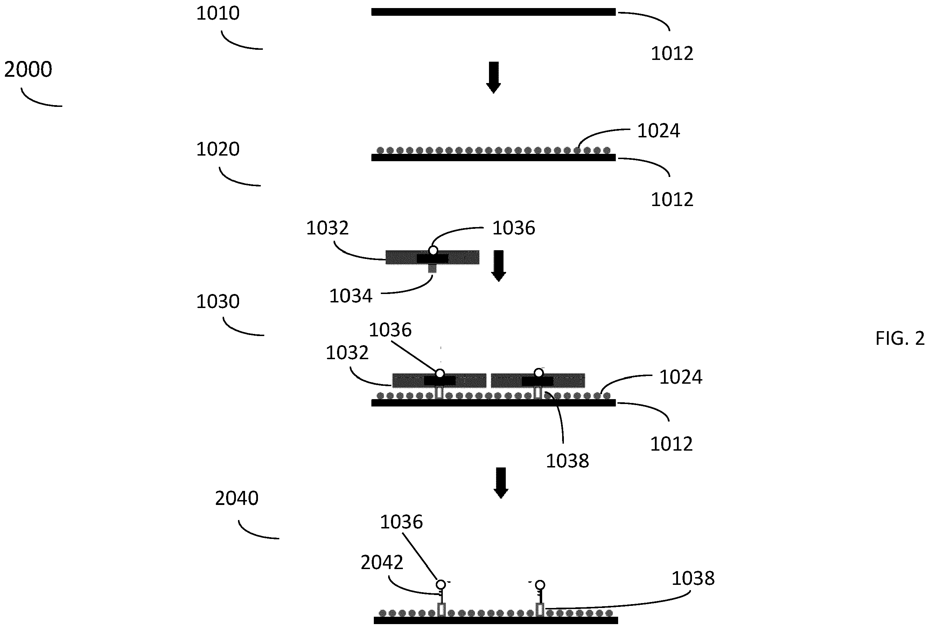

FIG. 2 depicts a schematic of a method 2000. The method 2000 includes the steps of the method 1000 as well as the step 2040. The nucleic acid nanostructures 1032 include a nucleic acid 2042 that includes the second moiety 1034 and the payload moiety 1036. In the step 2040, a portion of the nucleic acid nanostructure 1032 is removed, leaving the nucleic acid 2042 on the surface 1012. The positon of the nucleic acids 2042 and the payload moieties 1036 are constrained relative to the surface 1012. A minimum distance (e.g., at least 10 nm, at least 100 nm, at least 1000 nm) is maintained between the payload moieties 1036. The nucleic acids 2042 can include DNA and/or RNA.

The step 2040 can be achieved by dissociating, denaturing, enzymatically digesting, chemically degrading and/or physically degrading the nucleic acid nanostructures 1032.

In some embodiments, the step 2040 involves removing a solution that surrounds the nucleic acid nanostructures 1032 and adding a new solution. Adding a new solution increases the entropic cost associated with nucleic acid nanostructures 1032 remaining bound to the surface 1012 as the new solution would initially be free of nucleic acid nanostructures 1032. Additionally, or alternatively, the step 2040 can include greatly increasing the volume of the solution that surrounds the nucleic acid nanostructures 1032, thereby decreasing the concentration of nucleic acid nanostructures 1032 in solution and increasing the entropic cost of remaining bound to the surface 1012.

In certain embodiments, the step 2040 involves introducing nucleic acid strands (e.g., DNA strands) that displace nucleic acid strands maintaining the structure of the nucleic acid nanostructures 1032. For example, strands maintaining the structure of the nucleic acid nanostructure (e.g., staple strands) can include a toehold. Displacement strands complementary to the staple strands can be introduced and hybridize with the staple strands via the toehold, thereby dissociating the nucleic acid nanostructure 1032.

In some embodiments, the step 2040 involves introducing a DNA binding protein to dissociate the structure of the nucleic acid nanostructures 1032. For example, the DNA binding protein can be transcription factors, polymerases, nucleases, histones, zinc finger domain-containing proteins, RPA, TAL effectors. By displacing hybridized or nearby DNA strands from other DNA strands, these proteins could destabilize the nucleic acid nanostructures 1032 leading to its removal from the surface 1012.

In certain embodiments, the step 2040 involves cleaving nucleic acid strands in the nucleic acid nanostructures 1032. In certain embodiments, the nucleic acid strands in the nucleic acid nanostructures 1032 can be cleaved using a nuclease (e.g. a restriction enzyme, BAL-31 Nuclease, Cas9 Endonuclease, DNase I Endonuclease, Exonuclease I, Exonuclease III, Exonuclease T, Exonuclease V, Exonuclease VII, Exonuclease VIII (truncated), Lambda Exonuclease, Mung Bean Nuclease, Nuclease P1, RecJf, T5 Exonuclease, T7 Exonuclease).

In some embodiments, the step 2040 involves denaturing the nucleic acid nanostructures 1032, for example, by reducing an ion concentration in the solution, changing a pH of the solution, adding DMSO, adding urea, and/or increasing the temperature. In some embodiments, changing the pH of the solution involves adding a base (e.g., NaOH) to increase the pH of the solution.

In certain embodiments, the step 2040 involves chemical degradation of the nucleic acid nanostructures 1032. Chemical degradation can be achieved by using an acid (e.g. trichloroacetic acid, formic acid, sulfuric acid, bisulfite), a base (hydrazine, Nba(OH)₂ + NaOH), a redox reaction (e.g. Mn(II)-blemycin with H₂O₂, Fe(II) or Fe(III)-blemycin + H₂O₂ or 2-mercaptoethanol, Mn(II)-cytochrome P-450), and/or nucleophilic addition (e.g. formamide).

In some embodiments, the step 2040 involves physical degradation of the nucleic acid nanostructures 1032. Physical degradation can be achieved using high temperatures, plasma and/or photoablation. High temperatures include the use of O₂, N₂, low humidity and high humidity atmospheres.

### Flexible linkers

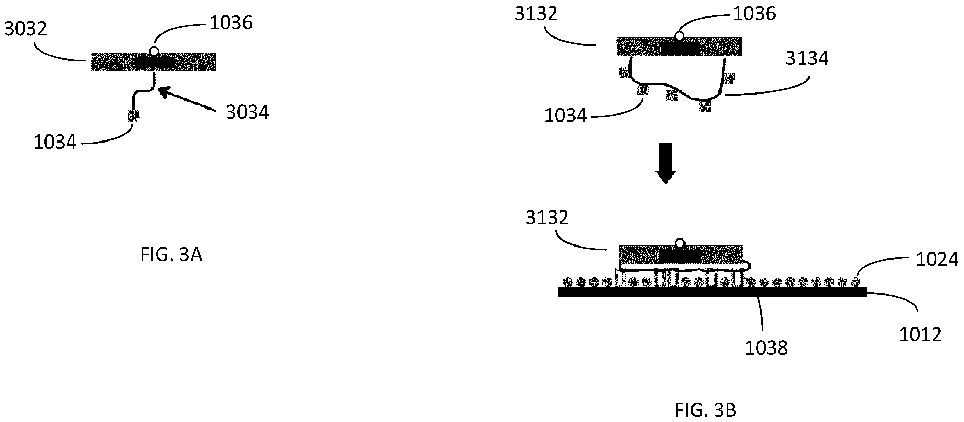

FIG 3A depicts a nucleic acid nanostructure 3032 that can be used in a method of the disclosure (e.g., the method 1000, the method 2000 and/or a method described below). The nucleic acid nanostructure 3032 includes the payload moiety 1036 and a flexible linker 3034 that includes the second moiety 1034. The flexible linker 3034 is a flexible structure that provides a number of geometric arrangements in which second moiety 1034 can interact with first moieties 1024 on surface 1012. For example, the flexible linker 3034 can be a chain-like strand that can flex to bring the second moiety 1034 near to first moieties 1024 without nucleic acid nanostructure 3032 necessarily being aligned relative to surface 1012. To this end, at least a portion the flexible linker 3034 can freely rotate relative to the shaped nucleic acid nanostructure 3032.

Examples of the flexible linker 3034 include PEG, a single stranded nucleic acid (e.g., single stranded DNA) and a double stranded nucleic acid (e.g., double stranded DNA). In some embodiments, the flexible linker 3034 has a length of at least 0.5 (e.g., at least 1, at least 5, at least 10, at least 15, at least 20, at least 25, at least 30, at least 35, at least 40, at least 45) nm and/or at most 50 (e.g., at most 45, at most 40, at most 35, at most 30, at most 25, at most 20, at most 15, at most 10, at most 5, at most 1) nm.

FIG. 3B depicts a nucleic acid nanostructure 3132 that can be used in a method of the disclosure (e.g., the method 1000, the method 2000 and/or a method described below) unbound and bound to a surface 1012 functionalized with first moieties 1024. The nucleic acid nanostructure 3132 includes the payload moiety 1036 and a flexible linker 3134 that includes a plurality of second moieties 1034. In some embodiments, the flexible linker 3134 includes a single attachment point to the nucleic acid nanostructure 3132. In some embodiments, the flexible linker 3134 includes two attachment points to the nucleic acid nanostructure 3132. When the nucleic acid nanostructure 3132 is disposed on the surface 1012 functionalized with the first moieties 1024, at least a portion (e.g., all) of the plurality of second moieties 1034 of the flexible linker 3134 can form complementary interactions 1038 (e.g., a covalent bond, a ligand-receptor interaction) with the first moieties 1024. In certain embodiments, the flexible linker 3134 includes at least 1 (e.g., at least 2, at leas 3, at least 4, at least 5, at least 10, at least 20, at least 30, at least 50) and/or at most 100 (e.g., at most 50, at most 30, at most 20, at most 10, at most 5, at most 4, at most 3, at most 2) second moieties 1034.

Without wishing to be bound by theory, it is believed that sequential formation of the complementary interactions 1038 can sequentially reduce the variability of the location and orientation of the nucleic acid nanostructures 3132 relative to the surface 1012 as successive first moieties 1024 and second moieties 1034 interact.

### Cleavable groups

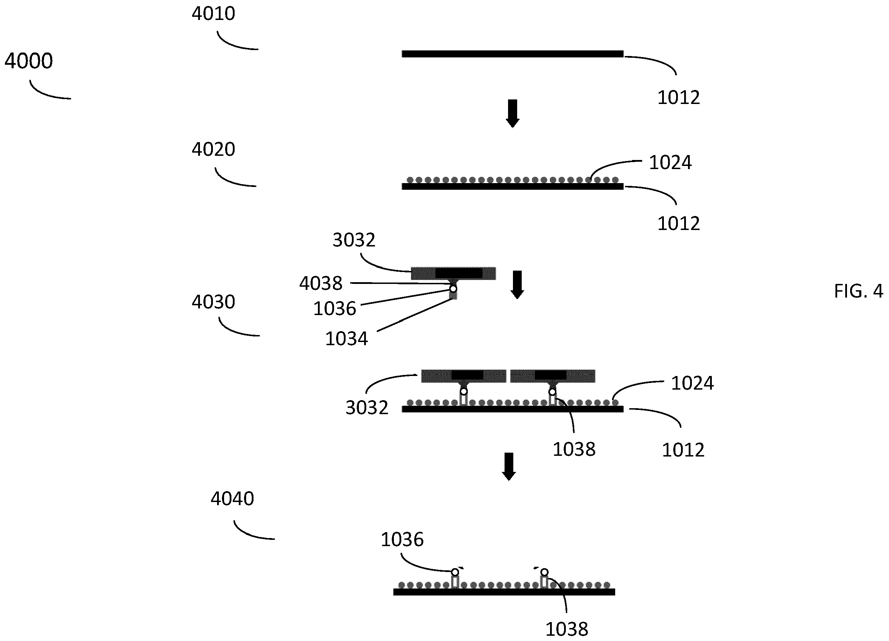

FIG. 4 depicts a schematic for a method 4000. In step 4010, a surface 1012 is provided. In step 4020 the surface 1012 is functionalized with the first moiety 1024. In step 4030 a plurality of nucleic acid nanostructures 4032 are disposed on the surface 1012. The nucleic acid nanostructures 4032 include the second moiety 1034, the payload moiety 1036 and a group 4038. The second moiety 1034 and the payload moiety 1036 are attached to the nucleic acid nanostructure 4032 via the group 4038. The first moieties 1024 and second moieties 1034 form a complementary interaction 1038 (e.g., a covalent bond, a ligand-receptor interaction, a van der Waals interaction). In step 4040, the group 4038 is cleaved. The nucleic acid nanostructures 4032 dissociate from the surface 1012, leaving the payload moieties 1036 attached to the surface 1012. The complementary interactions between the first moieties 1024 and second moieties 1034 constrain the positions of the payload moieties 1036 relative to the surface 1012. A minimum distance (e.g., at least 10 nm, at least 100 nm, at least 1000 nm) is maintained between the payload moieties 1036.

The second moiety 1034, the payload moiety 1036 and the group 4038 can be directly attached to one another or attached to one another via a linker group, e.g. an ether (e.g. PEG), an ester, a thioester, an amide, an imine, a carbamate, an anhydride, an aliphatic group, an alkene, an aromatic group, DNA, a peptide, and/or a carbohydrate linker.

The group 4038 can include a photocleavable group, a heat cleavable group, an ultrasound cleavable group, a chemically cleavable group, a pH labile group, a thiol based linker and/or an enzyme cleavable group.

Examples of photocleavable groups include a group cleaved by UV-light (e.g. *ortho-*Nitrobenzene, *ortho*-Nitrobenzofuran, *ortho*-Nitromandelic acid, *ortho*-Nitrophenylehtyl, Thioacetal *ortho*-nitrobenzene, Coumarin, Benzoin, Carbazole, *ortho*-Hydroxy cinnamate, Quinoline, Xanthene); a group cleaved by visible light, which induces a ROS mediated mechanism (e.g. cyanines); and a group cleaved via upconversion nanocrystals (e.g. *ortho-*Nitrobenzene, Thioacetal *ortho*-nitrobenzene, Coumarin).

Examples of heat cleavable groups include azo-based linkers (e.g. azobis[N-(2-carboxyethyl)-2-methylpropionamidine]), and gold thiol bonds (Au-S-R).

Examples of ultrasound cleavable groups include a combination of hemaotporphorin monomethyl ether and rose bengal to produce reactive oxygen species by activation with ultrasound and light.

Examples of chemically cleavable groups include diazo-based linkers that can be cleaved by sodium dithionite; ester-based, hydrazone-based or sulfonamide-based linkers that can be cleaved by nucleophilic attacks such as by hydroxylamine; malondialdehyde-indole based linkers that can be cleaved by pyrrolidine; and nitrobenzenesulfonamide linkers that can be cleaved by BME (beta-mercaptoethanol).

Examples of pH labile groups include carbamate-based linkers that are cleavable with trifluoroacetic acid; hydrazone-based linkers (e.g. Acylhydrazone) and diphenyldialkoxysilane that are cleavable under acidic conditions; and ester-based linkers that are cleavable under basic conditions.

Examples of thiol-based linkers include disulfide-bridges that are cleavable by Dithiothreitol, TCEP, BME and/or glutathion.

Examples of enzyme cleavable groups include amide and peptide based linkers that are cleavable by peptidases and/or proteases, such as lysine or argnine-based linkers that are cleavable by trypsin.

### Multiple payload moieties

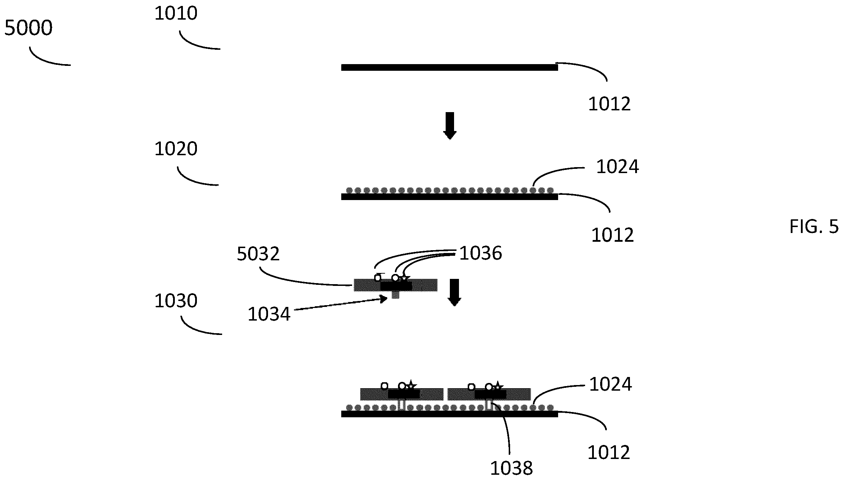

FIG. 5 depicts a schematic of a method 5000. The method 5000 includes the steps of the method 1000. However, in the step 1030 a plurality of nucleic acid nanostructure 5032 are disposed on the surface 1012. Each nucleic acid nanostructure 5032 includes a plurality of payload moieties 1036. The identities of payload moieties 1036 can be the same or different on a given nucleic acid nanostructure 5032. The position of each payload moiety on the nucleic acid nanostructure 5032, the distance between payload moieties 1036 on the nucleic acid nanostructure 5032 and the number of payload moieties 1036 per nucleic acid nanostructure 5032 can be controlled. In some embodiments, the number of payload moieties 1036 per nucleic acid nanostructure is at least 2 (e.g., at least 3, at least 4, at least 5). In some embodiments, the distance between payload moieties 1036 on a nucleic acid nanostructure 5032 is at least 0 (e.g., at least 1, at least 2, at least 3, at least 5, at least 10, at least 20, at least 50, at least 100, at least 200) nm and/or at most 500 (e.g., at most 200, at most 100, at most 50, at most 20, at most 10, at most 5, at most 3, at most 2, at most 1) nm.

Combinations of multiple payload moieties 1036 can be, for example, combinations of different orthogonal chemical linker groups allowing the attachment of further different moieties in a controlled pattern in a later step; combinations of identical chemical linker groups arranged in specific patterns allowing the attachment of further moieties in a controlled pattern in a later step; combinations of fluorophores and/or electric-field-enhancing-payloads such as metal nanoparticles; combinations of payloads that can bind specifically to target moieties (e.g. groups of antibodies binding to different epitopes of a target moiety); combinations of payloads that contain moieties that perform a function and moieties that control the rate of the function (e.g. a polymerase and a second enzyme that catalyzes a moiety that controls the rate of the polymerase activity); and/or combinations of payloads that bind specifically to target moieties and payloads that act on target moieties (e.g. antibodies and proteases, polymerases, nucleases, and/or phosphatases).

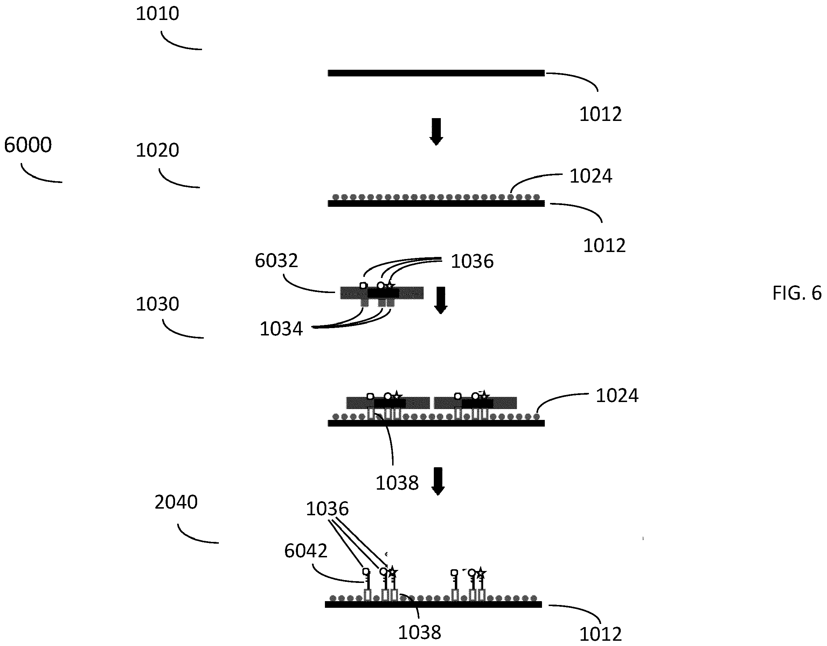

FIG. 6 depicts a schematic of a method 6000. The method 6000 includes the steps of the method 2000. However, in the step 1030 a plurality of nucleic acid nanostructures 6032 are disposed on the surface 1012. Each nucleic acid nanostructure 6032 includes a plurality of payload moieties 1036 and a plurality of second moieties 1034. In some embodiments, the number of payload moieties 1036 is equal to the number of second moieties 1034 on each nucleic acid nanostructure 6032. The identities of payload moieties 1036 can be the same or different on a given nucleic acid nanostructure 6032. The location of and distance between payload moieties 1036 on a nucleic acid nanostructure 6032 and the number of payload moieties 1036 per nucleic acid nanostructure 6032 can be controlled. In some implementations, payload moieties 1036 and second moieties 1034 are generally bound to opposite sides of nucleic acid nanostructures 6032 such that when second moieties 1034 interact with first moieties 1024 at a surface, payload moieties 1036 are sterically accessible from the other side of nucleic acid nanostructures 6032. In other implementations, payload moieties 1036 are bound within nucleic acid nanostructures 1032 and sterically inaccessible.

The nucleic acid nanostructures 6032 further includes a plurality nucleic acids 6042 that each includes a second moiety 1034 and a payload moiety 1036. In the step 2040, a portion of the nucleic acid nanostructure 6032 is removed leaving the plurality of nucleic acids 6042 on the surface 1012. The positon of the nucleic acids 6042 and the payload moieties 1036 are constrained relative to the surface 1012. A minimum distance (e.g., at least 10 nm, at least 100 nm, at least 1000 nm) is maintained between the payload moieties 1036 deposited by different nucleic acid nanostructures 1032. The nucleic acids 6042 can be DNA, RNA and/or an unnatural nucleic acid (see discussion below). In some embodiments, the second moieties 1034 and the payload moieties 1036 are attached to the nucleic acid nanostructure 4032 via the group 4038 (see discussion above).

### Surfaces with depressions

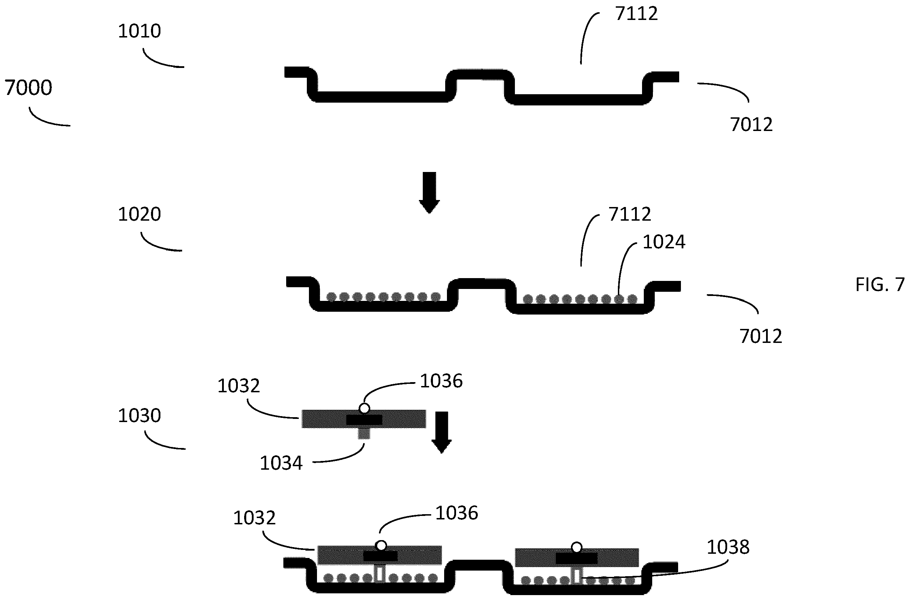

FIG. 7 depicts a schematic of a method 7000. The method 7000 includes the steps of the method 1000. However, the method uses a surface 7012 rather than the surface 1012. The surface 7012 defines a plurality of depressions 7112. The depressions 7112 are generally dimensioned to such that only one nucleic acid nanostructure 1032 can be received in a depression 7112. For example, when nucleic acid nanostructures 1032 are generally cylindrical structures with a smaller height dimension and larger length and width dimensions, depressions 7112 can be dimensioned such that their length and width dimensions are 150% or less of the length and width dimensions of nucleic acid nanostructures 1032. In general, the height of the depressions 7112 can be any appropriate height. The height of the depressions 7112 can be larger or smaller than a height dimension the nucleic acid nanostructures 1032.

In implementations in which nucleic acid nanostructures 1032 do not have cylindrical shapes, depression 7112 can be correspondingly modified to allow only one nucleic acid nanostructure 1032 to be received per depression 7112. For example, the nucleic acid nanostructures 1032 and the depressions 7112 can be rectangular, triangular, rod-shaped, cross-shaped, star-shaped, crescent-shaped, L-shaped, half-sphere-shaped, or conical.

In step 1020, the depressions 7112 of the surface 7012 are functionalized with the first moiety 1024. In step 1030, when the nucleic acid nanostructures 1032 are disposed on the surface 7012, the positions of the nucleic acid nanostructures 1032 and the payload moieties 1036 will be constrained to the depressions 7112. The size of the depressions can be configured such that only one nucleic acid nanostructure 1032 is included in a depression 7112. In such a configuration, the number of payload moieties 1036 included in a depression 7112 corresponds to the number of payload moieties per nucleic acid nanostructure 1032. For example, if each nucleic acid nanostructure 1032 includes a single payload moiety 1036, at most one payload moiety will be included in a depression 7112.

In some embodiments, the size of the depression 7112 is at least 1 (e.g., at least 2, at least 3, at least 4, at least 5, at least 6, at least 7, at least 8, at least 9, at least 10, at least 15, at least 20, at least 30, at least 40, at least 50, at least 60, at least 70, at least 80, at least 90, at least 100, at least 120, at least 150, at least 200, at least 250, at least 300, at least 350, at least 400, at least 450, at least 500, at least 550, at least 600, at least 650, at least 700, at least 750, at least 800, at least 850, at least 900, at least 950) nm and/or at most 1000 (e.g., at most 950, at most 900, at most 850, at most 800, at most 750, at most 700, at most 650, at most 600, at most 550, at most 500, at most 450, at most 400, at most 350, at most 300, at most 250, at most 200, at most 150, at most 120, at most 100, at most 90, at most 80, at most 70, at most 60, at most 50, at most 40, at most 30, at most 20, at most 10, at most 9, at most 8, at most 7, at most 6, at most 5, at most 4, at most 3, at most 2) nm. The non-functionalized regions can be arbitrarily large as they serve to separate the depressions 7112.

Examples of the surface 7012 include glass, quartz, fused silica, silicon, silicon oxide, gold, aluminum, graphene, glass, and/or a polymer such as PDMS, PMMA, polystyrene, a cyclic olefin copolymer (COC), a polycarbonate, and/or a polypropylene polymer (e.g. a nylon membrane). In some embodiments, the surface 7012 is passivated to reduce non-specific interactions. In some embodiments, the passivation of the surface 7012 includes PEG chains, 3D polymer matrices, polymer lawns, and/or 3D hydrogels. In some implementations, depressions 7112 can be formed, e.g., using lithographic or other patterning techniques.

### Surfaces functionalized with nucleic acids

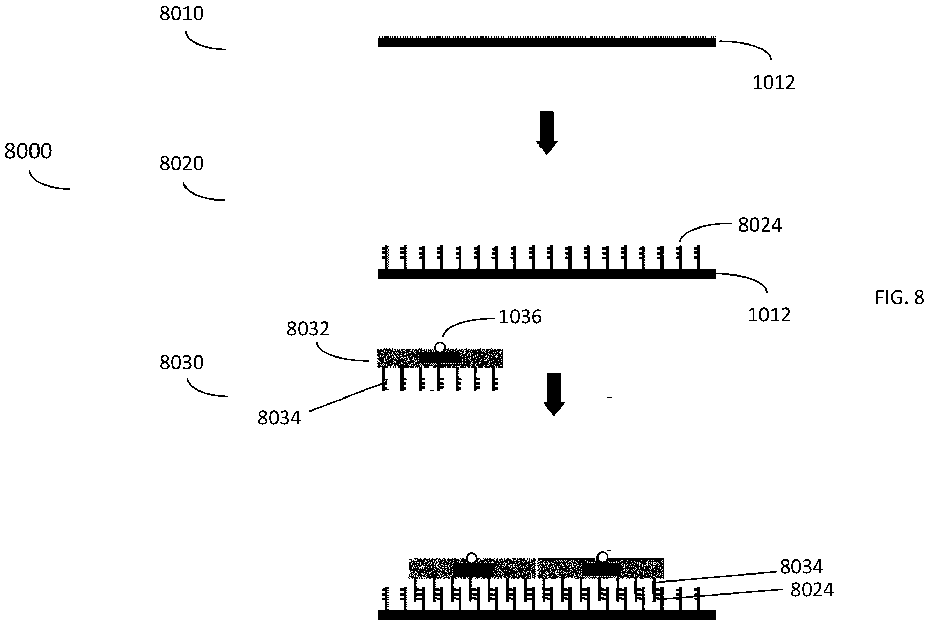

FIG. 8 depicts a schematic of a method 8000. In step 8010, a surface 1012 is provided. In step 8020, the surface 1012 is functionalized with a plurality of first nucleic acid strands 8024. In step 8030, a plurality of nucleic acid nanostructures 8032 are disposed on the surface 1012. The nucleic acid nanostructures 8032 include a plurality of second nucleic acid strands 8034 and a payload moiety 1036. A portion of the second nucleic acid strands 8034 is complementary with a portion of the first nucleic acid strands 8024. The first nucleic acid strands 8024 and the second nucleic acid strands 8034 hybridize and constrain the positions of the nucleic acid nanostructures 8032 and the payload moieties 1036 relative to the surface 1012. The hybridization of each second nucleic acid strand 8034 to the first nucleic acid strands 8024 should be relatively weak; thus, only the hybridization of multiple first nucleic acid strands 8024 and second nucleic acid strands 8034 leads to the binding of the nucleic acid nanostructures 8032 to the surface 1012. A minimum distance (e.g., at least 10 nm, at least 100 nm, at least 1000 nm) is maintained between the payload moieties 1036.

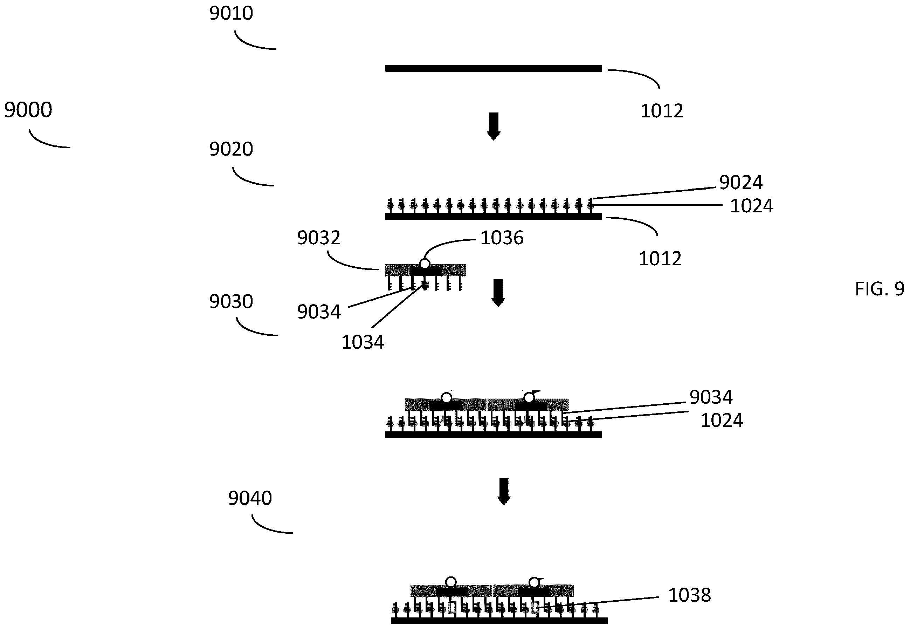

FIG. 9 depicts a schematic of a method 9000. In step 9010, a surface is provided. In step 9020, the surface is functionalized with a first nucleic acid strand 9024. At least a portion (e.g., all) of the first nucleic acid strands 9024 are functionalized with the first moiety 1024.

In step 9030, a plurality of nucleic acid nanostructures 9032 are disposed on the surface 1012. The nucleic acid nanostructures 9032 include a plurality of second nucleic acid strand 9034 and a payload moiety 1036. At least one second nucleic acid strand 9034 per nucleic acid nanostructure 9032 is functionalized with the second moiety 1034. A portion of the second nucleic acid strands 9034 is complementary with a portion of the first nucleic acid strands 9024. Additionally, the second moieties 1034 are configured to interact complementarily with the first moieties 1024. The first nucleic acid strands 9024 and the second nucleic acid strand 9034 hybridize.

In step 9040, the first moieties 1024 and second moieties 1034 form a complementary interaction 1038 (e.g., a covalent bond). The complementary interactions between the first moieties 1024 and second moieties 1034 constrain the positions of the nucleic acid nanostructures 9032 and the payload moieties 1036 relative to the surface 1012. A minimum distance (e.g., at least 10 nm, at least 100 nm, at least 1000 nm) is maintained between the payload moieties 1036.

Examples of the first nucleic acid strand 8024, the second nucleic acid strand 8034, the first nucleic acid strand 9024 and the second nucleic acid strand 9034 include DNA, RNA, locked nucleic acid (LNA), bridged nucleic acid (BNA), glycol nuclei acid (GNA), therose nuelci acid (TNA), peptide nucleic acid (PNA) and phosphorodiamidate morpholino oligomers (PMO).

### Surface passivation

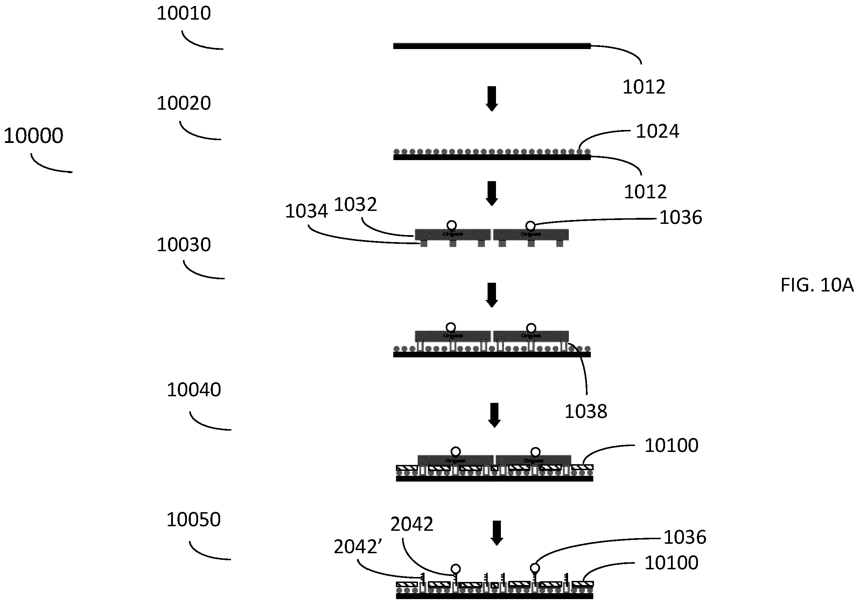

FIG. 10A depicts a schematic of a method 10000. In step 10010, a surface 1012 is provided. In step 10020, the surface 1012 is functionalized with the first moiety 1024. In step 10030, a plurality of nucleic acid nanostructures 1032 are disposed on the surface 1012. The nucleic acid nanostructures 1032 include one or more second moieties 1034 and the payload moiety 1036. The first moieties 1024 and second moieties 1034 can form a complementary interaction 1038 (e.g., a covalent bond, a ligand-receptor interaction). The complementary interactions between the first moieties 1024 and second moieties 1034 constrains the positions of the nucleic acid nanostructures 1032 and the payload moieties 1036 relative to the surface 1012. In the step 10040, the surface 1012 is functionalized with a passivation layer 10100. The passivation layer 10100 is disposed over the first moieties 1024 where complementary interactions 1038 have not formed to reduce (e.g., prevent) further reactions with the first moieties 1024 and/or reduce (e.g., prevent) interactions of other, subsequently introduced, molecules (e.g., molecules from a blood sample, a nucleic acid from a sequencing sample) with the surface 1012. The passivation layer 10100 attaches to the surface 1012 by reacting with the first moieties 1024. Examples of the passivation layer 10100 includes PEG, phospholipids, carboxybetaine, silicone, hydrogels, and/or polymer brushes.

The nucleic acid nanostructures 1032 include a nucleic acid 2042 that includes the second moiety 1034 and the payload moiety 1036. The nucleic acid nanostructures 1032 further include a nucleic acid 2042' that includes the second moiety 1034. In the step 10050, a portion of the nucleic acid nanostructures 1032 are removed leaving the nucleic acids 2042 and moieties on the surface 1012. The positon of the nucleic acids 2042 and 2042' and the payload moieties 1036 are constrained relative to the surface 1012. A minimum distance (e.g., at least 10 nm, at least 100 nm, at least 1000 nm) is maintained between the payload moieties 1036. In certain embodiments, the nucleic acid nanostructures 1032 do not include the nucleic acids 2042'.

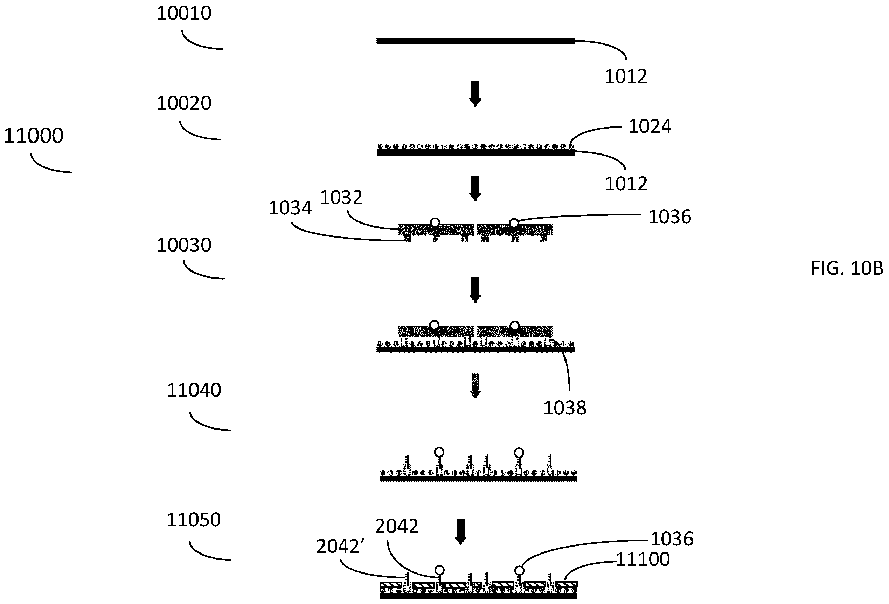

FIG. 10B depicts a schematic of a method 11000. The method 11000 includes the steps of the method 10000. However, rather than including the steps 10040 and 10050, the method 11000 includes the steps 11040 and 11050. In the step 11040, a portion of the nucleic acid nanostructures 1032 are removed leaving the nucleic acids 2042 and 2042' on the surface 1012. The positon of the nucleic acids 2042 and 2042' and the payload moieties 1036 are constrained relative to the surface 1012. A minimum distance (e.g., at least 10 nm, at least 100 nm, at least 1000 nm) is maintained between the payload moieties 1036. In the step 11050, the surface 1012 is functionalized with a passivation layer 11100. The passivation layer 11100 is disposed over the first moieties 1024 where complementary interactions 1038 have not formed to reduce (e.g., prevent) further reactions with the first moieties 1024 and/or reduce (e.g., prevent) interactions of other, subsequently introduced, molecules (e.g., molecules from a blood sample, a nucleic acid from a sequencing sample) with the surface 1012. Examples of the passivation layer 11100 includes PEG, phospholipids, carboxybetaine, silicone, hydrogels, and/or polymer brushes.

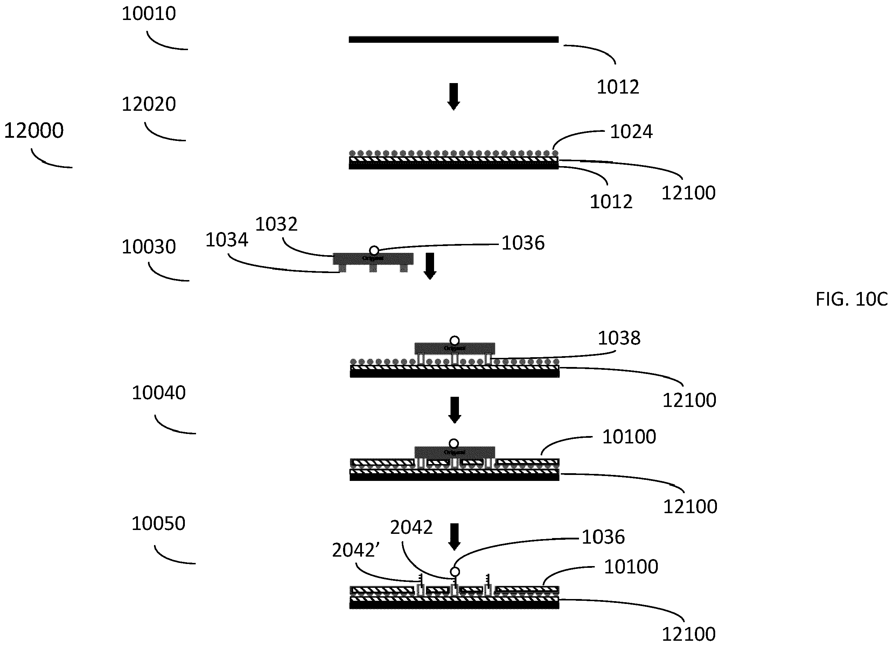

FIG. 10C depicts a schematic of a method 12000. The method 12000 includes the steps of the method 10000. However, rather than including the step 10020, the method 12000 includes the step 12020. In the step 12020, the surface 1012 is functionalized with a passivation layer 12100 and the first moieties 1024. The passivation layer 12100 supports the first moieties 1024. Examples of the passivation layer 12100 includes PEG, phospholipids, carboxybetaine, silicone, hydrogels, and/or polymer brushes.

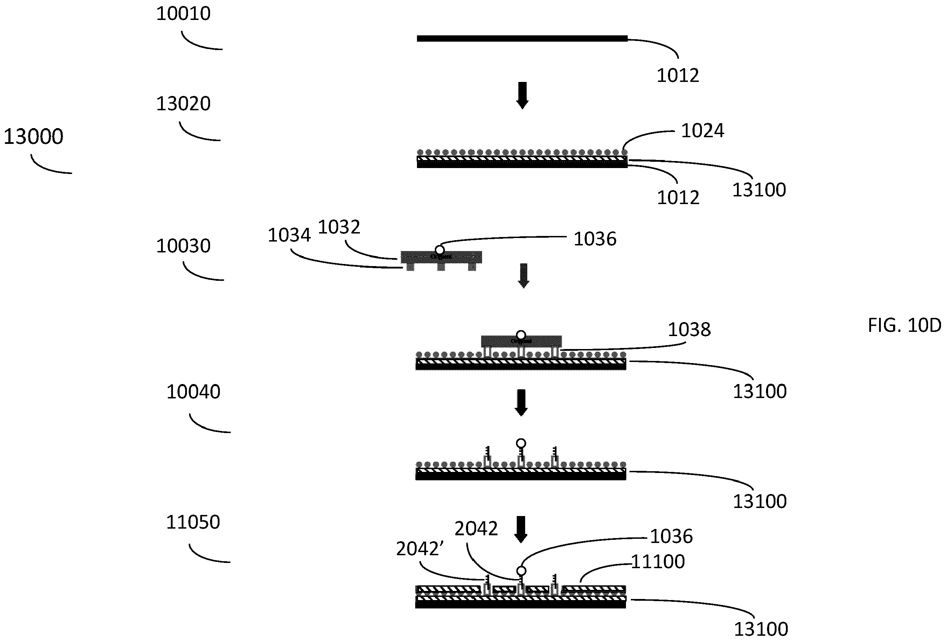

FIG. 10D depicts a schematic of a method 13000. The method 13000 includes the steps of the method 11000. However, rather than including the step 10020, the method 13000 includes the step 13020. In the step 13020, the surface 1012 is functionalized with a passivation layer 13100 and the first moieties 1024. The passivation layer 13100 supports the first moieties 1024. Examples of the passivation layer 13100 includes PEG, phospholipids, carboxybetaine, silicone, hydrogels, and/or polymer brushes.

### Passivation of nucleic acid nanostructures

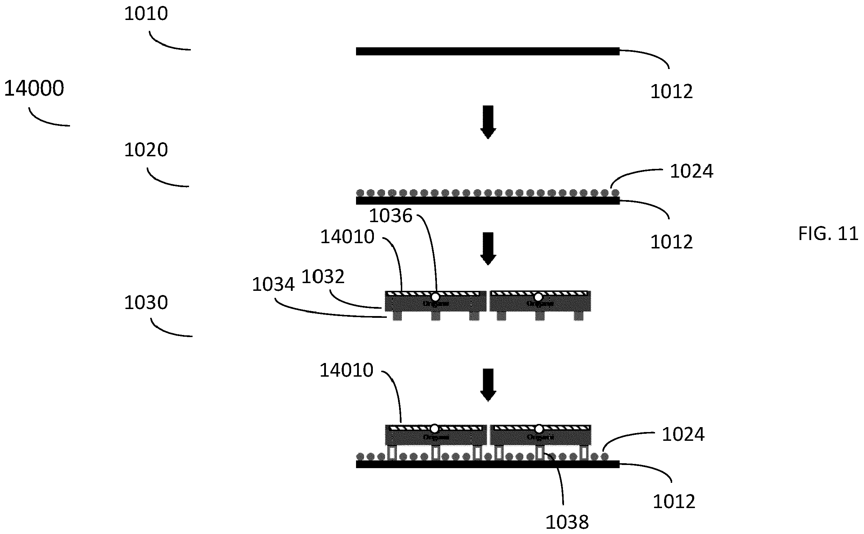

FIG. 11 depicts a schematic of a method 14000. The method 14000 includes the steps of the method 1000. A portion of each of the nucleic acid nanostructures 1032 introduced in the step 1030 includes a passivation layer 14010. Examples of the passivation layer 14010 include PEG, phospholipids, carboxybetaine, silicone, hydrogels, and/or polymer brushes.

Without wishing to be bound by theory, it is believed that the passivation layer 14010 can prevent unwanted binding of molecules to the surface 1012 at a later stage, such as molecules in a test sample to be analyzed (see discussion below).

### Use of first and second surfaces

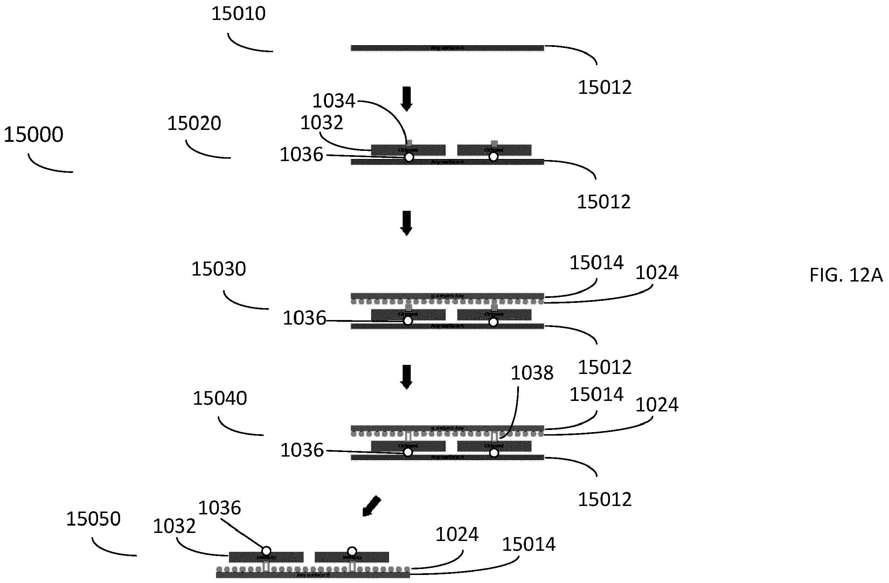

FIG. 12A depicts a schematic for a method 15000. In step 15010, a first surface 15012 is provided. For example, the first surface 15012 can be glass, quartz, fused silica, silicon, silicon oxide, gold, aluminum, graphene, glass, and/or a polymer. In step 15020, a plurality of nucleic acid nanostructures 1032 are disposed on the first surface 15012. The nucleic acid nanostructures 1032 include a second moiety 1034 and a payload moiety 1036. Disposing the nucleic acid nanostructures on the first surface 15012 in the step 15010 can include surface assisted binding, non-covalent interactions and/or covalent interactions.

In step 15030, a second surface 15014, functionalized with the first moiety 1024 is disposed on the nucleic acid nanostructures 1032 on the first surface 15012. For example, the second surface 15014 can be glass, quartz, fused silica, silicon, silicon oxide, gold, aluminum, graphene, glass, and/or a polymer. In step 15040, the first moieties 1024 and second moieties 1034 form a complementary interaction 1038 (e.g., a covalent bond, a ligand-receptor interaction). In step 15050, the first surface 15012 is removed leaving the nucleic acid nanostructures 1032 disposed on the second surface 15014. The complementary interactions between the first moieties 1024 and second moieties 1034 constrain the positions of the nucleic acid nanostructures 1032 and the payload moieties 1036 relative to the second surface 15014. A minimum distance (e.g., at least 10 nm, at least 100 nm, at least 1000 nm) is maintained between the payload moieties 1036.

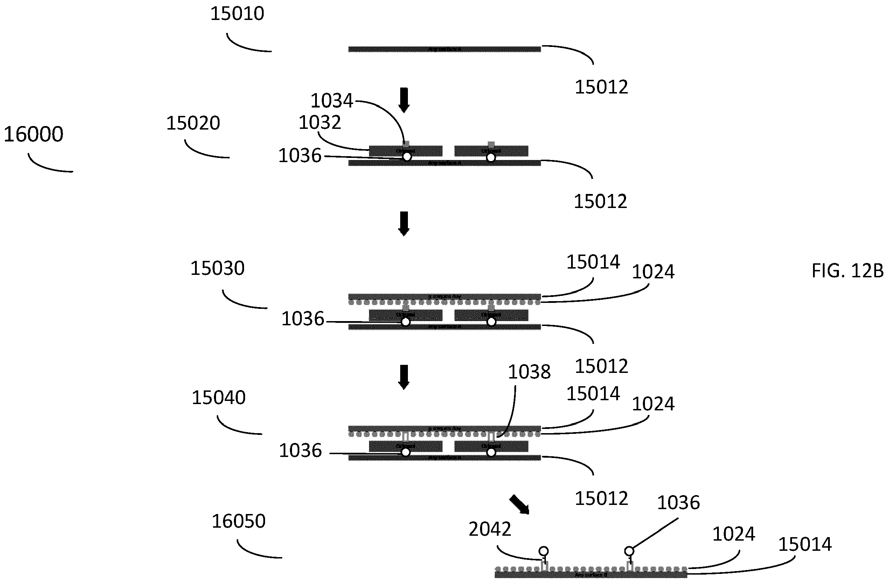

FIG. 12B depicts a schematic of a method 16000. The method 16000 includes the steps of the method 15000. However, rather than including the step 15050, the method 16000 includes the step 16050. In the step 16050, the first surface 15012 and a portion of the nucleic acid nanostructure 1032 are removed leaving the nucleic acids 2042 on the surface 1012. The positon of the nucleic acids 2042 and the payload moieties 1036 are constrained relative to the second surface 15014. A minimum distance (e.g., at least 10 nm, at least 100 nm, at least 1000 nm) is maintained between the payload moieties 1036.

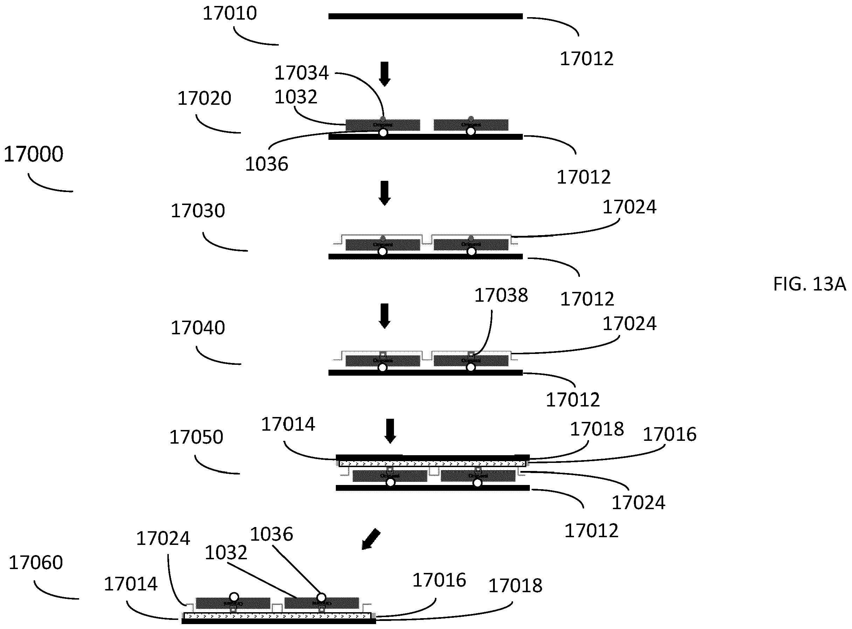

FIG. 13A depicts a schematic of a method 17000. In step 17010, a first surface 17012 is provided. In step 17020, a plurality of nucleic acid nanostructures 1032 are disposed on the first surface 17012. For example, the first surface 17012 can be glass, quartz, fused silica, silicon, silicon oxide, gold, aluminum, graphene, glass, and/or a polymer. The nucleic acid nanostructures 1032 include a second moiety 17034 and a payload moiety 1036. The second moiety 17034 includes a thiol group. Disposing the nucleic acid nanostructures on the first surface 17012 in the step 17010 can include surface assisted binding, non-covalent interactions and/or covalent interactions.

In step 17030, a layer of gold 17024 is deposited by sputtering on the first surface 17012 and the nucleic acid nanostructures 1032. In step 17040, the thiol group of the second moieties 17034 form thiol-gold bonds 17038 with the layer of gold 17024.

In step 17050, a second surface 17014 that includes a layer of PDMS 17016 and a layer of glass 17018 is disposed on the layer of gold 17024 such that the layer of PDMS 17016 contacts the layer of gold 17024. In step 17060, the first surface 17012 is removed leaving the nucleic acid nanostructures 1032 and layer of gold 17024 disposed on the layer of PDMS 17016 of the second surface 17014. The complementary interactions between the first moieties 17034 and the layer of gold 17024 constrain the positions of the nucleic acid nanostructures 1032 relative to the second surface 17014. A minimum distance (e.g., at least 10 nm, at least 100 nm, at least 1000 nm) is maintained between the payload moieties 1036. Other materials are possible in addition to the layer of PDMS 17016 and the layer of glass 17018.

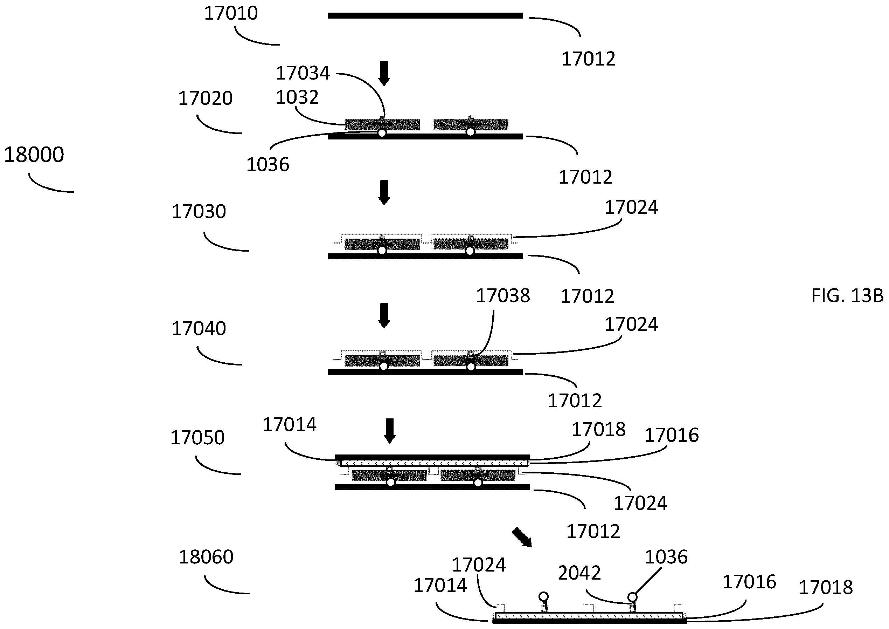

FIG. 13B depicts a schematic of a method 18000. The method 18000 includes the steps of the method 17000. However, rather than including the step 17060, the method 18000 includes the step 18060. In the step 18060, the first surface 17012 and a portion of the nucleic acid nanostructures 1032 are removed leaving the nucleic acids 2042 on the second surface 17014. The positon of the nucleic acids 2042 and the payload moieties 1036 are constrained relative to the second surface 17014. A minimum distance (e.g., at least 10 nm, at least 100 nm, at least 1000 nm) is maintained between the payload moieties 1036.

Examples of the first surface 15012, the second surface 15014, and the first surface 17012 include glass, quartz, fused silica, silicon, silicon oxide, gold, aluminum, graphene, glass, and/or a polymer such as PDMS, PMMA, polystyrene, a cyclic olefin copolymer (COC), a polycarbonate, and/or a polypropylene polymer (e.g. a nylon membrane). In some embodiments, the first surface 15012, the second surface 15014 and/or the first surface 17012 are passivated to reduce non-specific interactions. In some embodiments, the passivation of the first surface 15012, the second surface 15014 and/or the first surface 17012 includes PEG chains, 3D polymer matrices and/or 3D hydrogels. In some embodiments, first surface 15012, the second surface 15014, the first surface 17012 and/or the second surface 17014 is homogenous.

### Nucleic acid nanostructures

Generally, the shape and dimensions of a nucleic acid nanostructure of the disclosure (e.g., the nucleic acid nanostructure 1032, 3032, 3132, 5032, 6032, 8032, and/or 9032) can be varied as appropriate. Examples of the shapes of the nucleic acid nanostructures of the disclosure (e.g., the nucleic acid nanostructure 1032, 3032, 3132, 5032, 6032, 8032, and/or 9032) include circular, spherical, square, cuboidal, triangular, pyramidal, rod-like and ring-like structures.

The nucleic acid nanostructure of the disclosure (e.g., the nucleic acid nanostructure 1032, 3032, 3132, 5032, 6032, 8032, and/or 9032) can be constructed from any polynucleotide platform. Examples of polynucleotide platforms include DNA origami, RNA origami, hybrid DNA:RNA origami, DNA tiles, and structures containing locked nucleic acid (LNA), bridged nucleic acid (BNA), glycol nuclei acid (GNA), therose nuelci acid (TNA), peptide nucleic acid (PNA) and/or phosphorodiamidate morpholino oligomers (PMO).

In some embodiments, a nucleic acid of the disclosure (e.g., the nucleic acid nanostructure 1032, 3032, 3132, 5032, 6032, 8032, and/or 9032) includes a first face and a second face, the second moiety 1034 is disposed on the first face and the payload moiety 1036 is disposed on the second face.

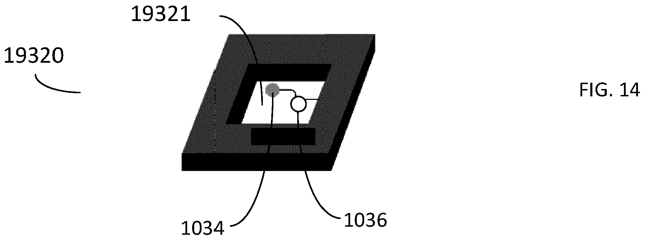

FIG. 14 depicts a schematic of a ring-shaped nucleic acid nanostructure 19320. The ring-shaped nucleic acid nanostructure 19320 defines a hole 19321. The second moiety 1034 and the payload moiety 1036 are contained in the hole 19321. Without wishing to be bound by theory, it is believed that the second moiety 1034 can react with a first moiety 1024 disposed on a surface (e.g., the surface 1012) independent of the side of the nucleic acid nanostructure 19320 that contacts the surface.

In some embodiments, in addition to interactions between the first moieties 1024 and the second moieties 1034, the nucleic acid nanostructures of the disclosure (e.g., the nucleic acid nanostructure 1032, 3032, 3132, 5032, 6032, and/or 9032) can include geometries with different non-covalent interaction strengths with a surface (e.g., the surface 1012).

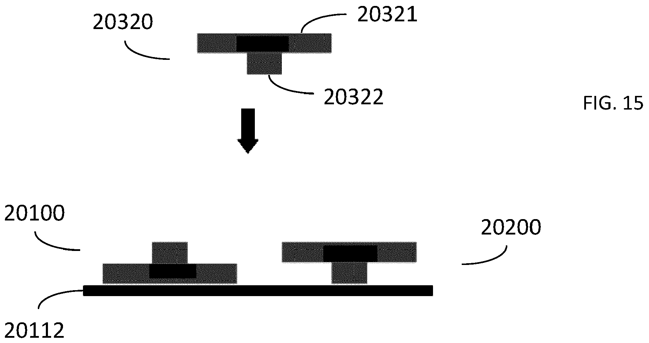

FIG. 15 depicts a schematic of nucleic acid nanostructure 20320, a first bound state 20100 to a surface 20112 and a second bound state 20200 to the surface 20112. The nucleic acid nanostructure 20320 includes a first side 20321 with a first geometry and a second side 20322 with a second geometry. The first side 20321 has a first interaction energy with the surface 20112 and the second side 20322 has a second interaction energy with the surface 20112 lower than the first interaction energy. Without wishing to be bound by theory, it is believed that the nucleic acid nanostructures 20320 will have a higher probability of binding the surface 20112 in the first bound state 20100 relative to the second bound state 20200 as the first bound state 20100 has a higher interaction energy with the surface 20122 relative to the second bound state 20200. Alternatively, the second interaction energy with the surface 20112 can be greater than the first interaction energy. In such a situation, the nucleic acid nanostructures 20320 will have a higher probability of binding the surface 20112 in the second bound state 20200 relative to the first bound state 20100.

In certain embodiments, in addition to interactions between the first moieties 1024 and the second moieties 1034, the nucleic acid nanostructures of the disclosure (e.g., the nucleic acid nanostructure 1032, 3032, 3132, 5032, 6032, and/or 9032) can include a surface modification.

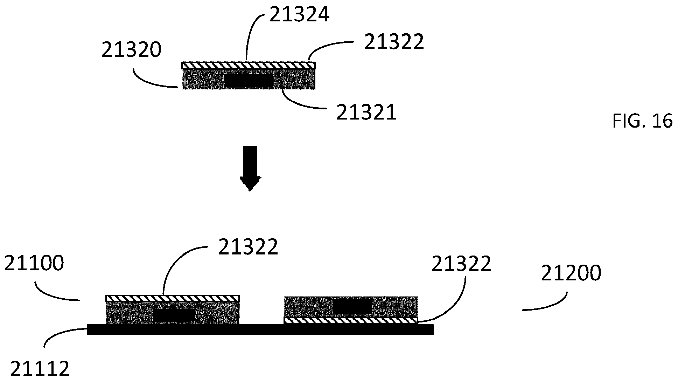

FIG. 16 depicts a schematic of nucleic acid nanostructure 21320, a first bound state 21100 to a surface 21112 and a second bound state 21200 to the surface 21112. The nucleic acid nanostructure 21320 includes a first side 21321 and a second side 21322. The second side 21322 includes a modification 21324. Examples of the modification 21324 include polyT single-stranded DNA, PEG and charged moieties.

The first side 21321 has a first interaction energy with the surface 21112 and the second side 21322 has a second interaction energy with the surface 21112 lower than the first interaction energy due to the presence of the modification 21324. Without wishing to be bound by theory, it is believed that the nucleic acid nanostructures 21320 will have a higher probability of binding the surface 21112 in the first bound state 21100 relative to the second bound state 21200 as the first bound state 21100 has a higher interaction energy with the surface 21122 relative to the second bound state 21200. Alternatively, the second interaction energy with the surface 21112 can be greater than the first interaction energy. In such a situation, the nucleic acid nanostructures 21320 will have a higher probability of binding the surface 21112 in the second bound state 21200 relative to the first bound state 21100.

In some embodiments, a nucleic acid nanostructure of the disclosure (e.g., the nucleic acid nanostructure 1032, 3032, 3132, 5032, 6032, 8032, 9032, 19320, 20320, and/or 21320) has a diameter, length and/or width of at least 10 (e.g., at least 20, at least 30, at least 40, at least 50, at least 60, at least 70, at least 80, at least 90, at least 100, at least 120, at least 150, at least 200, at least 250, at least 300, at least 350, at least 400, at least 450, at least 500, at least 550, at least 600, at least 650, at least 700, at least 750, at least 800, at least 850, at least 900, at least 950) nm and/or at most 1000 (e.g., at most 950, at most 900, at most 850, at most 800, at most 750, at most 700, at most 650, at most 600, at most 550, at most 500, at most 450, at most 400, at most 350, at most 300, at most 250, at most 200, at most 150, at most 120, at most 100, at most 90, at most 80, at most 70, at most 60, at most 50, at most 40, at most 30, at most 20) nm. In some embodiments, a nucleic acid nanostructure of the disclosure (e.g., the nucleic acid nanostructure 1032, 3032, 3132, 5032, 6032, 8032, 9032, 19320, 20320, and/or 21320) has a height of at least 2 (e.g., at least 3, at least 4, at least 5, at least 6, at least 7, at least 8, at least 9, at least 10, at least 20, at least 30, at least 40, at least 50, at least 60, at least 70, at least 80, at least 90, at least 100, at least 120, at least 150, at least 200, at least 250, at least 300, at least 350, at least 400, at least 450, at least 500, at least 550, at least 600, at least 650, at least 700, at least 750, at least 800, at least 850, at least 900, at least 950) nm and/or at most 1000 (e.g., at most 950, at most 900, at most 850, at most 800, at most 750, at most 700, at most 650, at most 600, at most 550, at most 500, at most 450, at most 400, at most 350, at most 300, at most 250, at most 200, at most 150, at most 120, at most 100, at most 90, at most 80, at most 70, at most 60, at most 50, at most 40, at most 30, at most 20, at most 10, at most 9, at most 8, at most 7, at most 6, at most 5, at most 4, at most 3) nm.

In some embodiments, a nucleic acid nanostructure of the disclosure (e.g., the nucleic acid nanostructure 1032, 3032, 3132, 5032, 6032, 8032, 9032, 19320, 20320, and/or 21320) has a ratio of diameter, length and/or width to height of at least 1:1 (e.g., at least 2:1, at least 3:1, at least 4:1, at least 5:1, at least 6:1, at least 7:1, at least 8:1, at least 9:1, at least 10:1, at least 20:1, at least 30:1, at least 40:1, at least 50:1, at least 60:1, at least 70:1, at least 80:1, at least 90:1, at least 100:1, at least 150:1, at least 200:1, at least 250:1, at least 300:1, at least 350:1, at least 400:1, at least 450:1) and/or at most 500:1 (e.g., at most 450:1, at most 400:1, at most 350:1, at most 300:1, at most 250:1, at most 200:1, at most 150:1, at most 100:1, at most 90:1, at most 80:1, at most 70:1, at most 60:1, at most 50:1, at most 40:1, at most 30:1, at most 20:1, at most 10:1, at most 9:1, at most 8:1, at most 7:1, at most 6:1, at most 5:1, at most 4:1, at most 3:1, at most 2:1).

### Systems that include nucleic acid nanostructures and/or payload moieties

The methods of the disclosure (e.g., the method 1000, 2000, 4000, 5000, 6000, 7000, 8000, 9000, 10000, 11000, 12000, 13000, 14000, 15000, 16000, 17000, and/or 18000) can be used to construct a system. Examples of the system include a biochip, a metasurface, a surface coating, a quantum computer and/or optoelectronics. The biochips can be used, for example in DNA sequencing, proteomics, protein analysis, protein sequencing, analyzing protein-interactions, data storage, and/or molecular sensing.

The system can be used in proteomics, sequencing, super-resolution microscopy, protein analytics, surface plasmon resonance (SPR), Bio-layer interferometry (BLI), switchSENSE, quantum computing, photonics, light harvesting, catalysts, nucleic acid data storage, and/or chemical sensing.

### Example

### Example 1

Coverslips were functionalized with azide functional groups (PolyAn, part number: 10400626). The coverslips were stored in a fridge. Prior to use, coverslips were given 15 minutes to adjust to ambient conditions before opening the box to avoid condensation on the coverslips. A multi-channel slide (Ibidi, Product number 80608) was attached to the coverslips, creating multiple channels.

DNA origami nanostructures with dimensions of 100x70x2 nm functionalized with 8 DBCO groups on a bottom side of the structure, and DNA strands adapted to bind an imager strand on a top side were dissolved in 1×TE buffer with 12.5 mM MgCl at 25 °C and 90 % RH to final concentrations of 10, 50, 100 and 1000 pM. 150 µL of diluted DBCO functionalized DNA origami nanostructure solution or buffer, as shown in Table 1, was added to a coverslip channel and allowed to incubate for 4 hours. All buffers listed in Table 1 included 12.5 mM MgCl. The volume remained constant over the incubation. After incubation, the channels were cleaned with 1×TE buffer once, 1×TE buffer with 0.1 % Tween 14 times and 1×TE buffer 3 times.

**Table 1: channel composition**

| Channel | Composition |
|---|---|
| 1 | 10 pM DNA origami nanostructures |
| 2 | 50 pM DNA origami nanostructures |
| 3 | 1×TE buffer |
| 4 | 100 pM DNA origami nanostructures |
| 5 | TE buffer |
| 6 | 1000 pM DNA origami nanostructures |

Imaging buffer was prepared by combining 1×TE buffer with 12.5 mM MgCl₂ and 2 µL Tween 20. The imaging buffer was vortexed until foam formed on top of the solution. Prior to imaging, the channel was washed once with 200 µL of imaging buffer. 170 µL of cy3b-modified imager strand in imaging buffer was added to the channel. The imager strand contained a fluorescent dye, and bound weakly and intermittently to the origami, allowing for super-resolution imaging.

A commercial Nikon MEA54000 Ti2-E inverse TIRF microscope equipped with a 100x TIRF objective (Nikon, MRD01991), a 561 nm laser with 30 mW output power (Nikon, DI20000 LightHUB+N Laser Engine) and CMOS camera (Hamamatsu, ORCA-Fusion Digital CMOS camera C14440-20UP) was used for imaging. The imaging parameters were 10,000 frames with a frame duration of 100 ms, and pixels were binned using 2x2 binning. Imaging and analysis using Picasso software were performed as described in Schnitzbauer, Joerg, et al. "Super-resolution microscopy with DNA-PAINT." Nature protocols 12.6 (2017): 1198-1228, which is hereby incorporated by reference.

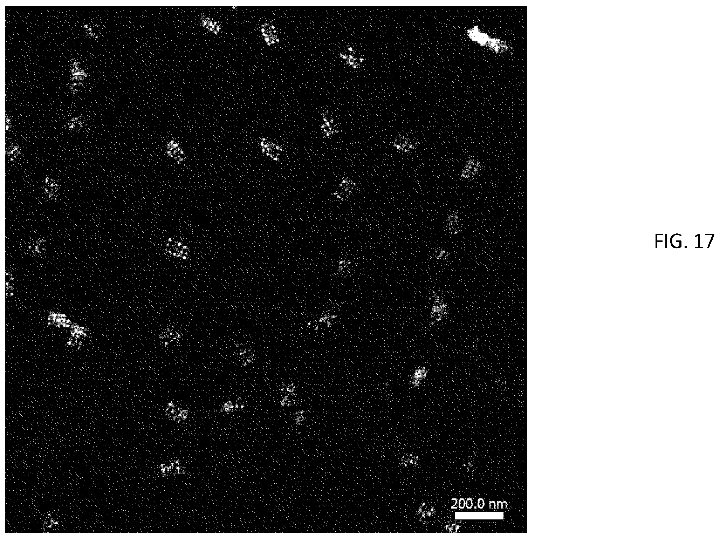

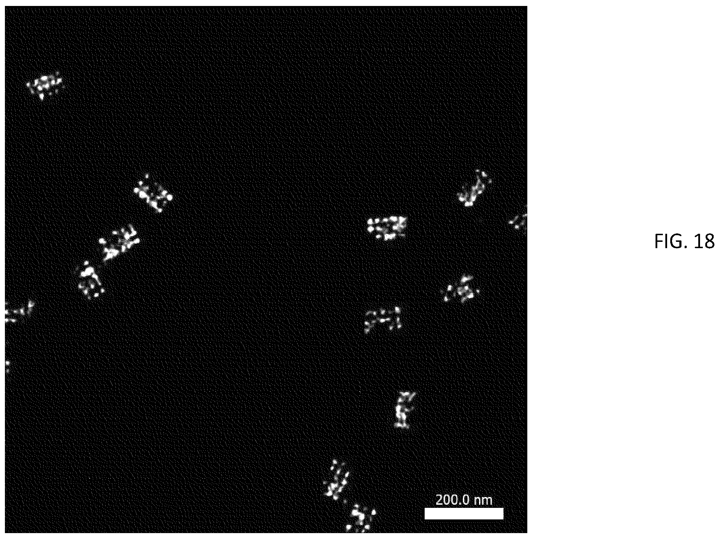

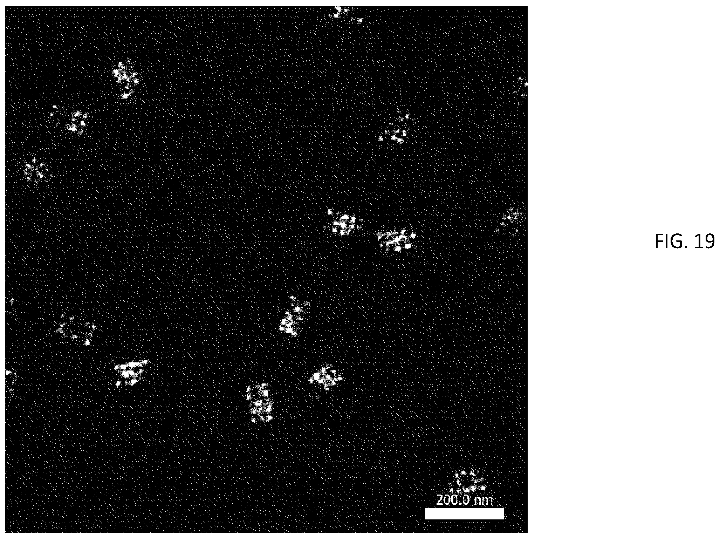

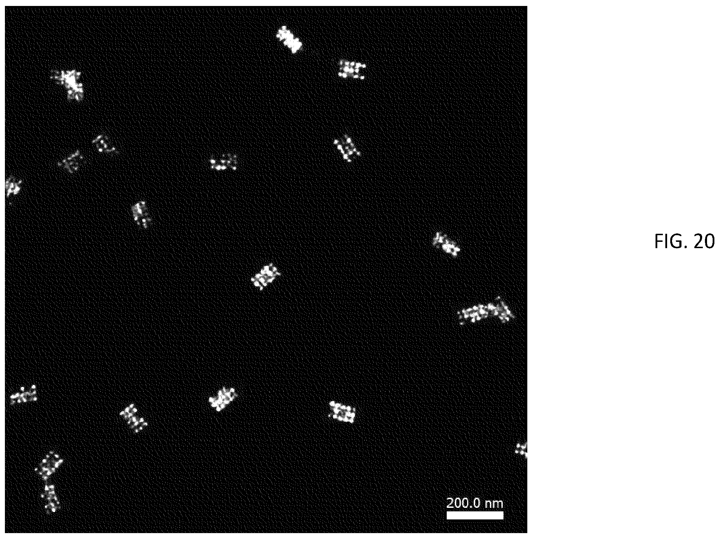

FIGS. 17-20 show fluorescent images of surfaces after placement of the DNA origami nanostructures. FIG. 17 is from the 50 pM nanostructure channel (channel 2) and FIGS. 18-20 are from the 100 pM nanostructure channel (channel 4). Clusters of bright spots represent individual DNA origami nanostructures covalently bound to the surface. The structures bound densely but did not overlap, maintaining a fixed minimal distance between each other.

The images demonstrate that DNA origami structures and hence a payload they bear can be disposed on a surface without overlap, providing a minimum distance between payload moieties of adjacent DNA origami structures.

### Example 2

Using the methods of Example 1, nanostructures with dimensions of 100x70x2 nm functionalized with DBCO groups on a bottom side of the structure, and 4 DNA strands adapted to bind an imager strand placed closely to one another on a top side were incubated for 4 hours at a concentration of 4 nM. The DNA strands adapted to bind an imager strand were placed close to one another to generate a single, relatively high intensity spot in super resolution DNA PAINT microscopy.

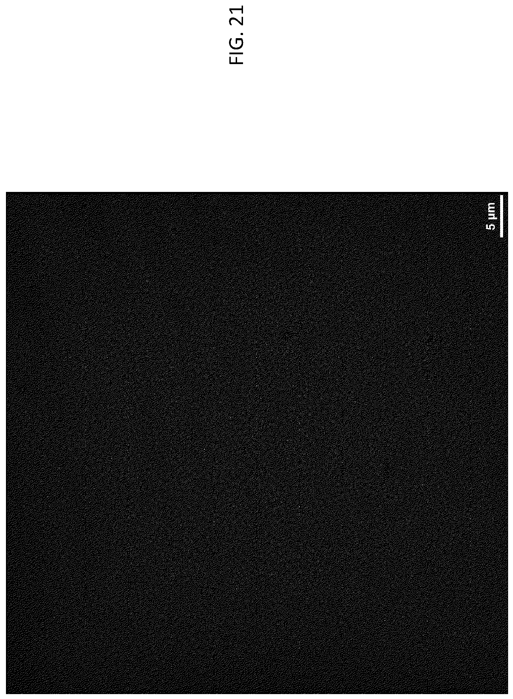

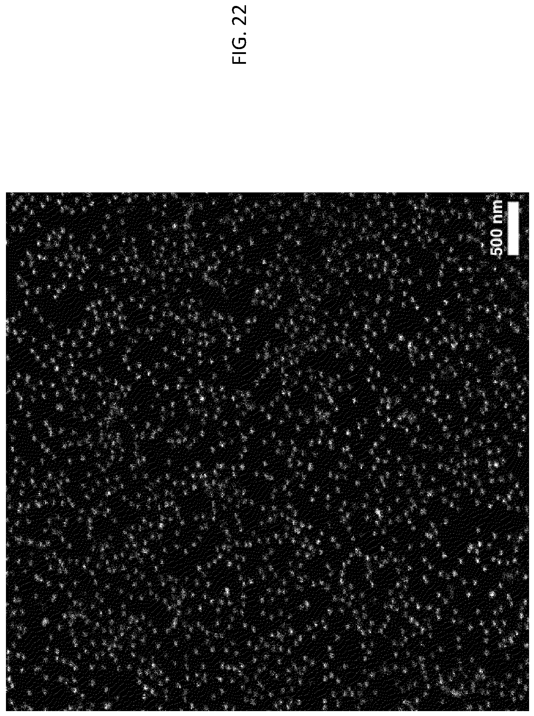

Samples were imaged as described in Example 1. FIG. 21 shows a fluorescent image of surfaces after placement of the DNA origami nanostructures. FIG. 22 shows a zoom in of a region in FIG. 21.

The raw image data was analyzed as described in Example 1 to generate localizations of binding sites. Individual structures were then identified by using the HDBSCAN algorithm to identify clusters of localizations. Using the center of mass of each cluster as the location for a structure, the distance to the nearest neighbor for each structure was calculated. From the distribution of nearest-neighbor distances, the probability not to observe another structure for a given distance was calculated. The probability of no other moieties being within a distance r from a placed moiety for a random poissonian process is exp( - r² * Pi * rho), with rho being the area density of moieties.

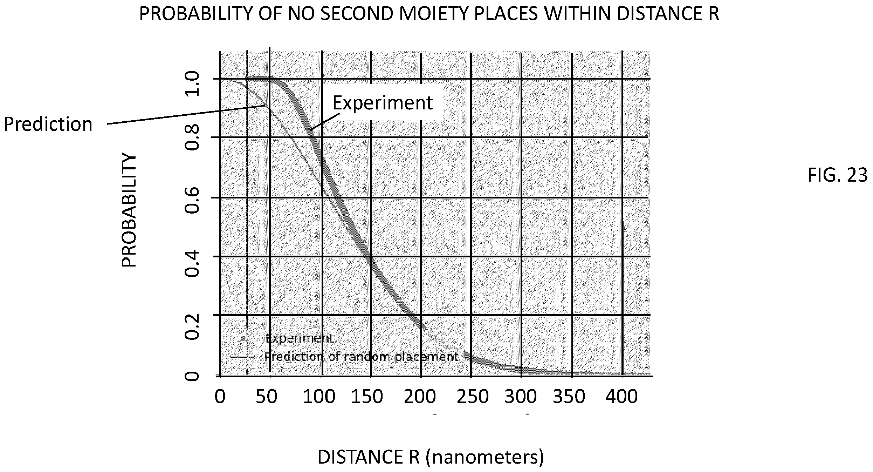

FIG. 23 shows a graph of the probability of a second payload moiety not being placed relative to a first payload moiety as a function of distance. The difference between the prediction of random placement and experimental results is due to the DNA origami nanostructures ensuring a minimum distance between payloads.

### Other Embodiments

While certain embodiments have been disclosed above, the disclosure is not limited to such embodiments.

As an example, while embodiments have been disclosure that include DNA and/or strands of DNA; the disclosure is not limited to such embodiments. In some embodiments, the nucleic acid nanostructures and/or nucleic acid strands of the disclosure include DNA, RNA, locked nucleic acid (LNA), bridged nucleic acid (BNA), glycol nuclei acid (GNA), therose nuelci acid (TNA), peptide nucleic acid (PNA) and/or phosphorodiamidate morpholino oligomers (PMO).

## Claims

1. A method comprising:
exposing a plurality of nucleic acid nanostructures to a surface functionalized with a plurality of first moieties, each of the nucleic acid nanostructures comprising
a payload moiety positioned at a site of each of the nucleic acid nanostructures and
a second moiety; and
forming either a covalent bond or a ligand-receptor interaction between the first moieties and the second moieties such that positions of the nucleic acid nanostructures and the payload moieties are constrained relative to the surface; and
wherein the sites of the payload moieties in the nucleic acid nanostructures are disposed such that a minimum distance is maintained between the payload moieties of each nucleic acid nanostructure when the first moieties covalently bond or interact via the ligand-receptor interaction with second moieties.

2. The method of claim 1, wherein:
the second moieties are coupled to a generally planar face of the nucleic acid nanostructures; and
the nucleic acid nanostructures have a height dimension extending perpendicularly from the generally planar face that is smaller than other dimensions of the nucleic acid nanostructures.

3. The method of claim 1 or 2, wherein:
the nucleic acid nanostructures comprise a length of 10 nm to 1000 nm; or
the nucleic acid nanostructures comprise a height of 2 nm to 1000 nm; or
a ratio of length to height of the nucleic acid nanostructures is 1:1 to 500:1; or
a height dimension of the nucleic acid nanostructures is less than 20% of the smallest of the other dimensions of the nucleic acid nanostructures.

4. The method of any one of claims 1-3, wherein the payload moieties are coupled to the nucleic acid nanostructures in a vicinity of a middle of the other dimensions of the nucleic acid nanostructures.

5. The method of any one of claims 1-4, wherein:
each nucleic acid nanostructures comprises a first face and a second face;
the second moiety is disposed on the first face; and
the payload moiety is disposed on the second face.

6. The method of any one of claims 1-5, wherein each nucleic acid nanostructure further comprises a first portion comprising a nucleic acid comprising the second moiety and the payload moiety, and
the method further comprises removing a second portion of each nucleic acid nanostructure from the surface,
wherein the second portion is different from the first portion.

7. The method of any one of claims 1-6, wherein each nucleic acid nanostructure comprises a cleavable group and the second moiety and the payload moiety are attached to the nucleic acid nanostructure via the cleavable group, and
the method further comprises cleaving the cleavable groups.

8. The method of any one of claims 1-7, wherein the nucleic acid nanostructures comprise a flexible linker and the second moiety is attached to the flexible linker.

9. The method of any one of claims 1-8, wherein:
the surface is flat; or
the surface is unpatterned; or
the surface comprises depressions functionalized with the first moiety and the depressions are configured such that at most one nucleic acid nanostructure can occupy each depression.

10. The method of any one of claims 1-9, wherein:
the payload moiety comprises a member selected from the group consisting of a fluorescent dye, a quencher, a quantum dot, a nanoparticle, a chemical linker group, a coordination complex, and a molecular switch; or
the payload moiety comprises a protein, for example, a protein selected from the group consisting of streptavidin, a streptavidin-like moiety, a polymerase, an antibody, horseradish peroxidase, a protein from a patient sample, a G protein, a G protein-coupled receptor (GPCRs), an ion channel, a cell-surface protein, and a receptor protein; or
the payload moiety comprises a nucleic acid, for example, a nucleic acid selected from the group consisting of DNA, RNA, a viral sequence, a human sequence, a bacterial sequence, an artificial sequence and/or a sequence with non-natural bases.

11. The method of any one of claims 1-10, wherein:
a minimum distance between payload moieties of each nucleic acid nanostructures is at least 10 nm; or
a minimum distance between payload moieties of each nucleic acid nanostructures is at least 100 nm; or
a minimum distance between payload moieties of each nucleic acid nanostructures is at least 1000 nm.

12. The method of any one of claims 1-11, wherein:
a density of the nucleic acid nanostructures is 1 to 10000 per µm²; or
a density of payload moieties is 1 to 1000000 per µm².

13. A system, comprising:
a surface functionalized with a plurality of first moieties; and
a plurality of nucleic acid nanostructures, each of the nucleic acid nanostructures comprising;
a payload moiety; and
a second moiety that covalently bonds or interacts via a ligand-receptor interaction with the first moieties;
wherein positions of the nucleic acid nanostructures and the payload moieties are constrained relative to the surface; and
wherein the nucleic acid nanostructures are configured on the surface such that a minimum distance is maintained between the payload moieties of each nucleic acid nanostructures.

14. A method comprising, performing at least one member selected from the group consisting of proteomics, sequencing, super-resolution microscopy, protein analytics, surface plasmon resonance (SPR), Bio-layer interferometry (BLI), switchSENSE, quantum computing, photonics, light harvesting, catalysts, nucleic acid data storage, and chemical sensing, using the system of claim 13.

15. The system of claim 13, formed by the method of any one of claims 1-12.
